# EUROPEAN PATENT SPECIFICATION

(11) **EP 1 769 085 B1**
(45) Date of publication and mention of the grant of the patent: **27.01.2010**
(21) Application number: 05759714.8
(22) Date of filing: 15.07.2005
(51) Int. Cl.: C12Q 1/68

(54) **DETECTION OF CELLULAR STRESS using Hmox-1 promoter**
NACHWEIS VON ZELLULÄREM STRESS mittels Hmox-1 Promoter
DETECTION DU STRESS CELLULAIRE en utilisant le promoteur de Hmox-1

(30) Priority: 16.07.2004 GB 0415963
(43) Date of publication of application: 04.04.2007
(73) Proprietor: CXR BIOSCIENCES LIMITED, Dundee DD1 5JJ (GB); ROSLIN INSTITUTE (EDINBURGH), Roslin Midlothian EH25 9PS (GB)
(72) Inventor: WOLF, Charles Roland, CXR Biosciences Limited, Dundee DD1 5JJ (GB); WHITELAW, Christopher B.A., Midlothian EH25 9PS (GB); ELCOMBE, Clifford Roy, CXR Biosciences Limited, Dundee DD1 5JJ (GB); BROWN, Kenneth, CXR Biosciences Limited, Dundee DD1 5JJ (GB); CLARK, Anthony, John, deceased (GB)
(74) Representative: Harrison Goddard Foote
(86) International application number: PCT/GB2005/002779
(87) International publication number: WO 2006/008484

(56) References cited:
- EP-A- 0 950 717
- WO-A-2004/011676
- US-A- 5 585 232
- US-A1- 2002 015 940
- US-A1- 2003 049 737
- SACCO MARIA GRAZIA ET AL: "Cell-based assay for the detection of chemically induced cellular stress by immortalized untransformed transgenic hepatocytes." BMC BIOTECHNOLOGY [ELECTRONIC RESOURCE]. 19 MAR 2004, vol. 4, 19 March 2004 (2004-03-19), page 5, XP002354484 ISSN: 1472-6750
- MALSTROM S E ET AL: "In vivo bioluminescent monitoring of chemical toxicity using heme oxygenase-luciferase transgenic mice" TOXICOLOGY AND APPLIED PHARMACOLOGY, ACADEMIC PRESS, vol. 200, no. 3, 15 July 2004 (2004-07-15), pages 219-228, XP004609284 ISSN: 0041-008X
- GARTEL A L ET AL: "Transcriptional regulation of the p21(waf1/cip1) gene" EXPERIMENTAL CELL RESEARCH, SAN DIEGO, CA, US, vol. 246, 1999, pages 280-289, XP002972657 ISSN: 0014-4827
- NAYLOR LH: "Reporter gene technology: The future looks bright" BIOCHEMICAL PHARMACOLOGY, PERGAMON, OXFORD, GB, vol. 58, 1999, pages 749-757, XP002902679 ISSN: 0006-2952

## Description

The present invention relates to a reporter gene construct, transfected cell lines including the construct and transgenic non-human animal models including the construct, the reporter gene construct incorporates nucleic acid sequences from a promoter region of a gene or set of genes whose expression is modified in response to external or internal changes in the cellular environment and in particular to stress conditions, the promoter being operatively linked to nucleic acid sequences chosen on the basis of the ease with which their transcription and/or translation products may be assayed. The reporter gene construct thereby provides a system capable of detecting intracellular conditions characteristic of biochemical stress or toxic conditions. In particular the present invention provides a reporter gene construct comprising, for example and without limitation, the Hmax-1 the promoter region for the construct.

### BACKGROUND

Contemporary methods of drug discovery, such as high-throughput screening of chemical libraries, identify large numbers of chemical entities with potential therapeutic effects. Only a fraction of these compounds will ever make useful drugs, however, because many of them have metabolic or toxicological characteristics that render them unsuitable for clinical use. Given the level of investment required to bring a potential new therapeutic drug to market, it is highly desirable that compounds possessing any adverse toxicological properties should be identified at an early stage, before significant development work has been undertaken.

A major problem with reliably screening large numbers of compounds for adverse toxicological characteristics is that methods currently available are either unsuitable for screening of large numbers of compounds or are too focused on specific mechanisms of toxicity to reliably predict the suitability of a given compound as a therapeutic drug. Thus, while *in vivo* pathological studies in experimental animals can give a very reliable indication of potential toxicity, the expense in terms of time and animal numbers required make such studies uneconomic for screening large numbers of compounds. The alternative to *in vivo* pathological studies is to employ *in vitro* methods of detecting known biochemical mechanisms. These are certainly amenable to high-throughput screening applications, but are limited in their ability to predict *in vivo* toxicity for a variety of reasons. These include the fact that: (*i*) only a selection of *in vivo* tissues can be represented cell culture systems; (*ii*) mechanisms of indirect toxicity seen *in vivo* are not replicated because effects of inter-organ interaction are absent in culture; (*iii*) the proliferation/quiescence state of cell lines or cells in primary culture is often different from their counterparts *in vivo* so that their responses are different; (*iv*) clonal selection of cell lines makes the results critically dependent on the particular cell line used.

A system that could monitor whole body indicators of toxicity in a manner that is economic for application to high-throughput screening would represent a very significant improvement over existing methods.

A cellular stress mechanism may be considered as the response of any gene or set of genes whose expression is modified in a cell, group of cells or tissue type in response to an external or internal change in the cellular environment that actually or potentially poses a threat to the normal functioning of the cell, neighbouring cells, tissues or the whole organism. Examples of changes in the cellular environment and genes whose expression may be transcriptionally modified as a result include:
1. Disturbances in the homeostatic state of DNA in the cell. These disturbances may include chemical alteration of nucleic acids or precursor nucleotides, inhibition of DNA synthesis and inhibition of DNA replication or damage to DNA. Genes whose expression may be altered in response to this type of disturbance include c-myc (Hoffman et al Oncogene 21 3414-3421), p21/WAF-1 (El-Diery Curr. Top. Microbiol. Immunol. 227 121-137 (1998); El-Diery Cell Death Differ. 8 1066-1075 (2001); Dotto Biochim. Biophys. Acta 1471 43-56 (2000)), MDM2 (Alarcon-Vargas & Ronai Carcinogenesis 23 541-547 (2002); Deb & Front Bioscience 7 235-243 (2002)), Gadd45 (Sheikh et al Biochem. Pharmacol. 59 43-45 (2000)), FasL (Wajant Science 296 1635-1636 (2002)), GAHSP40 (Hamajima et al J. Cell. Biol. 84 401-407 (2002)), TRAIL-R2/DR5 (Wu et al Adv.Exp. Med. Biol. 465 143-151 (2000); El-Diery Cell Death Differ. 8 1066-1075 (2001)), BTG2/PC3 (Tirone et al J. Cell. Physiol. 187 155-165 (2001));
2. Changes in the oxidative status of the cell. Such changes may be brought about by e.g. accumulation of free radicals or hypoxia. Genes whose expression may be altered in response to changes of this type include MnSOD and/or CuZnSOD (Halliwell Free Radic. Res. 31 261-272 (1999); Gutteridge & Halliwell Ann. NY Acad. Sci. 899 136-147 (2000)), I□B (Ghosh & Karin Cell 109 Suppl.., S81-96 (2002)), ATF4 (Hai & Hartman Gene 273 1-11 (2001)), xanthine oxidase (Pristos Chem. Biol. Interact. 129 195-208 (2000)), COX2 (Hinz & Brune J. Pharmacol. Exp. Ther. 300 376-375 (2002)), iNOS (Alderton et al Biochem. J. 357 593-615 (2001)), Ets-2 (Bartel et al Oncogene 19 6443-6454 (2000)), FasL/CD95L (Wajant Science 296 1635-1636 (2002)), □GCS (Lu Curr. Top. Cell. Regul. 36 95-116 (2000); Soltaninassab et al J. Cell. Physiol. 182 163-170 (2000)), ORP150 (Ozawa et al Cancer Res. 61 4206-4213 (2001); Ozawa et al J. Biol. Chem. 274 6397-6404 (1999)).
3. Changes that cause hepatotoxic stress. Genes whose expression may be altered in response to hepatotoxic stress may include Lrg-21 (Drysdale et al Mol. Immunol. 33 989-998 (1996)), SOCS-2 and/or SOCS-3 (Tollet-Egnell et al Endocrinol. 140 3693-3704 (1999), PAI-1 (Fink et al Cell. Physiol. Biochem. 11 105-114 (2001)), GBP28/adiponectin (Yoda-Murakami et al Biochem. Biophys. Res. Commun. 285 372-377 (2001)), □-1 acid glycoprotein (Komori et al Biochem Pharmacol. 62 1391-1397 (2001)), metallothioneine I (Palmiter et al Mol. Cell. Biol. 13 5266-5275 (1993)), metallothioneine II (Schlager & Hart App. Toxicol. 20 395-405 (2000)), ATF3 (Hai & Hartman Gene 273 1-11 (2001)), IGFbp-3 (Popovici et al J. Clin. Endocrinol. Metab. 86 2653-2639 (2001)), VDGF (Ido et al Cancer Res. 61 3016-3021 (2001)) and HIF1□Tacchini et al Biochem. Pharmacol. 63 139-148 (2002).
4. Stimuli that could trigger the cell to go into apoptosis. Examples of genes whose expression may modified by pro-apoptotic stress are Gadd 34 (Hollander et al J. Biol. Chem. 272 13731-13737 (1997)), GAHSP40 (Hamajima et al J. Cell. Biol. 84 401-407 (2002)), TRAIL-R2/DR5 (Wu et al Adv.Exp. Med. Biol. 465 143-151 (2000); El-Diery Cell Death Differ. 8 1066-1075 (2001)), c-fos (Teng Int. Rev. Cytol. 197 137-202 (2000)), CHOP/Gadd153 (Talukder et al Oncogene 21 4280-4300 (2002)), APAF-1 (Cecconi & Gruss Cell. Mol. Life Sci. 5 1688-1698 (2001)), Gadd45 (Sheikh et al Biochem. Pharmacol. 59 43-45 (2000(), BTG2/PC3 (Tirone J. Cell. Physiol. 187 155-165 (2001)), Peg3/Pwl (Relaix et al Proc. Nat'l Acad. Sci. USA 97 2105-2110 (2000)), Siah 1a (Maeda et al FEBS Lett. 512 223-226 (2002)), S29 ribosomal protein (Khanna et al Biochem. Biophys. Res. Commun. 277 476-486 (2000)), FasL/CD95L (Wajant Science 296 1635-1636 (2002)), tissue tranglutaminase (Chen & Mehta Int. J. Cell. Biol. 31 817-836 (1999)), GRP78 (Rao et al FEBS Lett. 514 122-128 (2002)), Nur77/NGFI-B (Winoto Int. Arch. Allergy Immunol. 105 344-346 (1994)), CyclophilinD (Andreeva et al Int. J. Exp. Pathol. 80 305-315 (1999)), p73 (Yang et al Trends Genet. 18 90-95 (2002)) and Bak (Lutz Biochem. Soc. Trans. 28 51-56 (2000)).
5. Administration of chemicals, drugs or other xenobiotic agents . Examples of genes whose expression may be altered under such conditions are xenobiotic metabolising cytochrome p450 enzymes from the 2A, 2B, 2C, 2D, 2E, 2S, 3A, 4A and 4B gene families (Smith et al Xenobiotica 28 1129-1165 (1998); Honkaski & Negishi J. Biochem. Mol. Toxicol. 12 3-9 (1998); Raucy et al J. Pharmacol. Exp. Ther. 302 475-482 (2002); Quattrochi & Guzelian Drug Metab. Dispos. 29 615-622 (2001)).
6. Disease states either natural, modelled or induced. These diseases can be selected from but not limited to the list comprised of obesity, compromised immunity, degenerative neurological disorders, cancer, cardiovascular, inflammatory diseases, genetic diseases or metabolic disorders.

The p21 gene (also known variously as Cdkn-1a, WAF1, CIP1, SDI1 and MDA-6) encodes a protein whose cellular functions include inhibition of cyclin-dependent kinase and consequent cell cycle arrest and/or apoptosis (el-Deiry, W.S et al., Cell 75: 817-825, 1993; el-Deiry, W.S. et al., Cancer Res. 54: 1169-1174, 1994; Gartel, A.L. and Tyner, A.L, Mol. Cancer Therapeutics 1: 639-649, 2002). Transcription of the p21 gene is subject to control by a variety of mechanisms associated with cellular stresses including regulation by the p53 tumour suppressor protein and other transcription factors such as Sp1, AP2, BRCA1, vitamin D3 receptor, retinoic acid receptor, C/EBPs, and STATs. Analysis of the p21 gene promoter region has identified a number of *cis*-acting sequence elements lying within 5 kilobases of the transcription start site through which these effects are mediated (Gartel, A.L. and Tyner, A.L, Exp. Cell Res. 246: 280-289, 1999).

Reporter gene systems incorporating sequences from the promoter region of the p21 gene have been used experimentally to demonstrate the ability of specific sequence elements to mediate the actions of specific transcription factors in regulating p21 expression in specific cell types (el-Deiry, W.S. et al., Cancer Res. 55: 2910-2919, 1995; Biggs, J.R. et al., J. Biol. Chem. 271: 901-906, 1996; Chin, Y.E. et al., Science 272: 719-722; Matsumura, I. et al., Mol. Cell Biol. 17: 2933-2943, 1997; Zeng, Y.X. et al., Nat. Genet. 15: 78-82, 1997, Bellido, T. et al., J. Biol. Chem. 273: 21137-21144, 1998; Moustakas, A and Kardassis, D., Proc. Natl. Acad. Sci. USA 95: 6733-6738, 1998; Biggs, J.R. and Kraft, A.S., J. Biol. Chem. 274: 36987-36994, 1999; Mitchell, K.O. and el-Deiry, W.S., Cell Growth Differ. 10: 223-230, 1999; Yang, W.L. et al., Mol. Cell Biol. Res. Commun. 1: 125-131, 1999; Zhang, W. et al., J. Biol. Chem. 275: 18391-18398, 2000; Gartel, A.L. et al., Proc. Natl. Acad. Sci. USA 98: 4510-4515, 2001; Gartel, A.L. et al., Oncol. Res. 13: 405-408, 2003). However, there has been no prior account of a reporter gene system designed to provide readout of the entire range of transcriptional responses of the p21 gene or of its use for screening the effects of administered compounds *in vivo.*

It is known from the prior art to use Ho1-luc (heme oxgenase-luciferase) transgenic mice for *in vivo* screening for compounds as a marker of toxicity that induce luciferase expression following injection of a luciferein substrate (Malstrom et al 2004). However, Malstrom et al report that the luciferase signal induced by doxorubicin was from the intestine, kidneys, stomach, spleen, gonads and some liver and that "no treatment-related findings were observed in heart". This is in sharp contrast to common general knowledge that the heart is a well-known site of doxorubicin toxicity (see Sun et al. 2001 and references therein). This observation suggests that the transgene used by Malstrom et al fails to detect potential toxicity in certain critical circumstances and so could give rise to false negatives.

Accordingly, a reporter system that could be regulated in a manner analogous to that of the endogenous gene and that would provide improved detection of cellular stress responses involving gene activation. We have found in the present invention that a reporter system incorporating for example the Hmax-1 gene or another stress responsive gene has the potential to provide an accurate and more sensitive means of detecting a wide variety of physiologically relevant cellular stresses and therefore satisfies the need for a means of early detection of many kinds of toxicity.

### BRIEF SUMMARY OF THE DISCLOSURE

In the present invention we have developed a system based on the properties of stress responsive promoter sequences, for example and without limitation the p21 gene promoter. We have found surprisingly that construction of a reporter gene comprising a suitable array of promoter elements from, for example the Hmox-1 promoter, driving expression of a suitable readout gene can be used in a transfected cell lines or a transgenic animal to provide a rapid *in vitro or in vivo* assay of a wide variety of toxic mechanisms. The reporter transgenes of the present invention provide a system in which biochemical events associated with a variety of cellular stress mechanisms prognostic of toxicity can be examined conveniently in a whole animal.

### STATEMENT OF THE INVENTION

According to a first aspect of the invention there is provided a nucleic acid construct comprising (*i*) a nucleic acid sequence of a promoter region of Hmox-1 comprising up to 16.5kb upstream and 8kb downstream from the transcription start site of a mammalian Hmox-1 gene and (*ii*) a nucleic acid sequence comprising a transcriptional start site and (*iii*) at least one or more nucleic acid sequence capable of acting as a template for a defined RNA transcript and providing a read-out in the form of an excretable protein and/or an agent capable of being detected histologically.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

It will be appreciated that the Reporter Sequence provides a read-out of the system into which the nucleic acid construct of the present invention has been incorporated so that nucleic acid sequence which acts as template for a defined RNA transcript may be in the form of an excretable protein, protein complexes or fragments, enzymes, enzymatic products or conjugates, primary, secondary or further metabolites and/or salts thereof, non-biological products that are released by direct or secondary effects on the expression of the reporter gene product, hormones or antibodies or the read-out may produce an agent which is not excretable from a cell but which is capable of being detected histologically, for example and without limitation LacZ products. It will also be appreciated that the nucleic acid constructs of the present invention may comprise more than one Reporter Sequence, for example it may contain two or more Reporter Sequences that express excretable read-out products or the nucleic acid may comprise a Reporter Sequence that expresses an agent capable of being detected histologically and an expressed excretable read-out product. The number of Reporter Sequences and variety thereof is not intended to limit the scope of the application.

Preferably, the promoter sequence of the gene has altered expression in response to any one or more of the following stress situations:
(i) disturbances in the homeostatic state of DNA in the cell;
(ii) changes in response to oxidative stress of a cell;
(iii) changes that cause hepatotoxic stress
(iv) stimuli that trigger cellular apoptosis
(v) administration of chemicals, therapeutics or other xenobiotic agents or;
(vi) disease states whether natural, induced or modelled.

It will be appreciated that a luciferase reporter system is not desirable as a read-out system as luciferase detection requires the animals to receive a luciferin substrate. Luciferase expression will therefore be detectable only in organs and tissues accessible to injected luciferin and only before the luciferin has been cleared from the body. Moreover, there is a possibility that luciferin may affect the toxicity or other responses to administered test compounds.

In addition, using a luciferase reporter system, the animal under study must be anaesthetised or restrained. The stress and/or effects of anaesthesia itself may affect responses to administered test compounds. In the UK, legal limits on experimental procedures on living animals make repeated general anaesthesia or withholding of food and water for more than 2 hours extremely difficult to justify and would severely limit the application of the luciferase reporter readout system. Accordingly, in the present invention the Reporter Sequence is selected from the aforementioned examples with a view to minimizing background stress effects which could give rise to false readings.

Preferably, the promoter sequence of the construct of the present invention is Hmox-1 and optionally further comprises an additional promoter selected from any one or more of the following genes comprising the group p21, metallothionein 1A, PUMA, Gclc, Cox2, Ki67, Stk6, Hsp, Ho-1, PIG3 and SOD2.

In one embodiment of the invention, the nucleic acid construct comprises (*i*) an additional nucleic acid sequence (the "Promoter Sequence") comprising up to 5000 base pairs of DNA from the region immediately 5' to the transcription start site of a mammalian p21 gene and (*ii*) a nucleic acid sequence (the "Transcription Start Sequence") comprising a transcriptional start site and (*iii*) a nucleic acid sequence (the "Reporter Sequence") capable as acting as template for a defined RNA transcript.

The Promoter Sequence may comprise sequences from within 5000 base pairs 5' of the transcription start site of any mammalian p21 gene (also known as Cdkn1a, comprising one or more individual selected sequence lengths each comprising one or more of the sequence intervals in the following list where the number ranges refer to the inclusive range of nucleotide base pair positions 5' to the transcription start site: 0-5; 15-20; 64-69; 77-82; 93-143; 157-162; 688-696; 765-779; 1194-1212; 1256-1270; 1920-1928; 2553-2561; 4228-4236.

The Transcription Start Sequence may be provided by including part or all of the first non-coding exon of the p21 gene or a minimal eukaryote consensus promoter that will direct transcription by eukaryotic polymerases when associated with functional promoter elements or transcription factor binding sites, for example the PhCMV*-1 promoter (Furth et al., Proc. Nat. Acad. Sci. USA 91: 9302-9306, 1994).

The Reporter Sequence will be selected to provide a convenient read out of gene expression through assay of either the transcript or the encoded translation product polypeptide. Preferably, transcription of the Reporter Sequence may be conveniently detected by any one or more of the following:
(1) assay of the RNA transcript of the Reporter Sequence by, for instance:
   (a) electrophoretic separation and blot hybridization
   (b) reverse transcription polymerase chain reaction (PCR);
   (c) *in situ* hybridization;
   The skilled person can select hybridization probes or PCR primer oligonucleotide sequences suitable for assay of any transcript to be assayed.
(2) assay of a polypeptide translation product of the Reporter Sequence by, for instance:
   (a) immunoassay in which the skilled person will know how to produce and select antibodies suitable for assay of any particular peptide translation product, for instance, a lipocalin (WO04011676A2) or SEAP (GB0322196A0) or the beta chain of human choriogonadotrophin with or without an additional epitope tag peptide sequence chosen to make the polypeptide translation product detectable by a suitable existing antibody by, for instance:
      (i) radioimmunoassay;
      (ii) enzyme-linked immunosorbent assay;
      (iii) electrophoretic separation and immunoblotting;
      (iv) immunohistochemistry;
   (b) enzymatic activity specific to the polypeptide, for instance assay of bacterial chloramphenicol acetyl transferase activity measured by acetylation of chloramphenicol (Gorman Mol. Cell. Biol. 2: 1044-1051, 1982) or beta-galactosidase (*lac*Z) activity by its ability to metabolise X-Gal;
   (c) fluorescent or luminescent properties of the polypeptide, for instance green fluorescent protein from *Aequoria victoria*;
   (d) interference by the polypeptide in a chemical or biochemical reaction, for instance:
      (i) by assay of increased stability or decreased turnover of a mRNA;
      (ii) by assay of increased stability or decreased turnover of another protein, for instance as a result of removal of polyubiquitination;
      (iii) increased accumulation of small molecule metabolites.

It will be appreciated that in some instance the reporter gene construct of the present invention may comprise the same Promoter Sequence but that the Reporter Sequence may be varied so that the construct may provide a number of different read-outs as a result of the same Promoter Sequence.

In a further embodiment of the invention the nucleic acid construct comprises (*i*) an additional nucleic acid sequence (the "Promoter Sequence") comprising a 26kb upstream segment of a mammalian metallothioneien 1A gene and (*ii*) a nucleic acid sequence (the "Transcription Start Sequence") comprising a transcriptional start site and (*iii*) a nucleic acid sequence (the "Reporter Sequence") capable as acting as template for a defined RNA transcript.

In a yet further embodiment of the invention the nucleic acid construct comprises (*i*) an additional nucleic acid sequence (the "Promoter Sequence") comprising up to 16kb of an upstream gene sequence and a 5kb segment of downstream sequence of a mammalian PUMA gene and (*ii*) a nucleic acid sequence (the "Transcription Start Sequence") comprising a transcriptional start site and (*iii*) a nucleic acid sequence (the "Reporter Sequence") capable as acting as template for a defined RNA transcript.

In a yet further embodiment of the invention the nucleic acid construct comprises (*i*) an additional nucleic acid sequence (the "Promoter Sequence") comprising up to 16kb of an upstream gene sequence of a mammalian Gclc gene and (*ii*) a nucleic acid sequence (the "Transcription Start Sequence") comprising a transcriptional start site and (*iii*) a nucleic acid sequence (the "Reporter Sequence") capable as acting as template for a defined RNA transcript.

In a yet further embodiment of the invention the nucleic acid construct comprises (*i*) an additional nucleic acid sequence (the "Promoter Sequence") comprising up to 3.5kb sequence upstream from the transcriptional start site and a further 3kb segment from the coding sequences of a mammalian cox 2 gene and (*ii*) a nucleic acid sequence (the "Transcription Start Sequence") comprising a transcriptional start site and (*iii*) a nucleic acid sequence (the "Reporter Sequence") capable as acting as template for a defined RNA transcript.

In a yet further embodiment of the invention the nucleic acid construct comprises (*i*) an additional nucleic acid sequence (the "Promoter Sequence") comprising up to 30kb of an upstream gene sequence of the transcriptional start of a mammalian Ki67 gene and (*ii*) a nucleic acid sequence (the "Transcription start Sequence") comprising a transcriptional start site and (*iii*) a nucleic acid sequence (the "Reporter Sequence") capable as acting as template for a defined RNA transcript.

In a yet further embodiment of the invention the nucleic acid construct comprises (*i*) an additional nucleic acid sequence (the "Promoter Sequence") comprising an E4TF1 motif (-87 to -78) and an Sp-1 site (-129 to -121) and optionally a tandem repressor element CDE/CHR(-53 to -49/-39 to -35) of a mammalian Stk6 gene and (*ii*) a nucleic acid sequence (the "Transcription Start Sequence") comprising a transcriptional start site and (*iii*) a nucleic acid sequence (the "Reporter Sequence") capable as acting as template for a defined RNA transcript.

In a yet further embodiment of the invention the nucleic acid construct comprises (*i*) an additional nucleic acid sequence (the "Promoter Sequence") comprising up to 5kb of 5' flanking region of a mammalian Hsp70 gene and (*ii*) a nucleic acid sequence (the "Transcription Start Sequence") comprising a transcriptional start site and (*iii*) a nucleic acid sequence (the "Reporter Sequence") capable as acting as template for a defined RNA transcript.

In a yet further embodiment of the invention the nucleic acid construct comprises (*i*) an additional nucleic acid sequence (the "Promoter Sequence") comprising p53 recognition sequence repeats of a mammalian PIG3 gene and (*ii*) a nucleic acid sequence (the "Transcription Start Sequence") comprising a transcriptional start site and (*iii*) a nucleic acid sequence (the "Reporter Sequence") capable as acting as template for a defined RNA transcript.

The invention discloses but does not claim a nucleic acid construct comprising a Promoter Sequence, as defined under the first aspect of the present invention, operatively isolated from a Transcription Start Sequence and a Reporter Sequence, as defined under the first aspect of the present invention, by a nucleotide sequence (the "Isolator Sequence") flanked by nucleic acid sequences recognised by a site specific recombinase, or by insertion such that it is inverted with respect to a Transcription Start Sequence and a Reporter Sequence, as defined under the first aspect of the present invention. The recombinase recognition sites are arranged in such a way that the Isolator Sequence is deleted or the inverted Promoter Sequence's orientation is reversed in the presence of the recombinase. The construct also comprises a nucleic acid sequence comprising a tissue-specific promoter operatively linked to a gene encoding the coding sequence for the site-specific recombinase.

This aspect allows for detecting Reporter Sequence expression in specified tissues only. By controlling the appropriate recombinase expression using a tissue-specifc promoter, the inducible gene construct will only be viable in those tissues in which the promoter is active. For example, by driving recombinase activity from a liver-specific promoter, only the liver will contain re-arranged reporter construct, and hence will the only tissue in which Reporter Sequence expression can occur.

The recombination event producing an active Reporter Sequence expression unit may therefore only take place in tissues where the recombinase is expressed. In this way the Reporter Sequence may only be expressed in specified tissue types where expression of the recombinase results in a functional transcription unit comprised of the inducible promoter linked to the promoter. Site-specific recombinase systems know to perform such a function include the bacteriophage P1 cre-lox and the bacterial FLIP systems. The site-specific recombinase sequences may therefore be two *lox*P sites of bacteriophage P1

The use of site-specific recombination systems to generate precisely defined deletions in cultured mammalian cells has been demonstrated. Gu et al. (Cell 73: 1155-1164, 1993) describe how a deletion in the immunoglobulin switch region in mouse ES cells was generated between two copies of the bacteriophage P1 *lox*P site by transient expression of the Cre site-specific recombinase, leaving a single *lox*P site. Similarly, yeast FLP recombinase has been used to precisely delete a selectable marker defined by recombinase target sites in mouse erythroleukemia cells (Fiering et al., Proc. Nat. Acad. Sci. USA 90: 8469-8473, 1993). The Cre *lox* system is exemplified below, but other site-specific recombinase systems could be used.

A construct used in the Cre *lox* system will usually have the following three functional elements:
1. The expression cassette;
2. A negative selectable marker (for instance *Herpes simplex* virus thymidine kinase (TK) gene) expressed under the control of a ubiquitously expressed promoter (for instance phosphoglycerate kinase (Soriano et al., Cell 64: 693-702, 1991); and
3. Two copies of the bacteriophage P1 site-specific recombination site *lox*P (Baubonis et al., Nuc. Acids. Res. 21: 2025-2029, 1993) located at either end of the DNA fragment.

This construct can be eliminated from host cells or cell lines containing it by means of site-specific recombination between the two *lox*P sites mediated by Cre recombinase protein which can be introduced into the cells by lipofection (Baubonis et al., Nuc. Acids Res. 21: 2025-2029, 1993). Cells which have deleted DNA between the two *lox*P sites are selected for loss of the TK gene (or other negative selectable marker) by growth in medium containing the appropriate drug (ganciclovir in the case of TK).

According to another aspect of the invention there is provided a host cell transfected with a nucleic acid construct according to any one of the previous aspects of the invention. The cell type is preferably of human or non-human mammalian origin but may also be of other animal, plant, yeast or bacterial origin.

According to another aspect of the invention, there is provided a transgenic non-human animal in which the cells of the non-human animal express the protein encoded by the nucleic acid construct according to any one of the previous aspects of the invention. The transgenic animal is preferably a mouse but may be another mammalian species, for example another rodent, for instance a rat or a guinea pig, or another species such as rabbit, or a canine or feline, or an ungulate species such as ovine, porcine, equine, caprine, bovine, or a non-mammalian animal species, for instance an avian (such as poultry, for instance chicken or turkey).

In embodiments of the invention relating to the preparation of a transfected host cell or a transgenic non-human animal comprising the use of a nucleic acid construct as previously described, the cell or non-human animal may be subjected to further transgenesis, in which the transgenesis is the introduction of an additional gene or genes or protein-encoding nucleic acid sequence or sequences. The transgenesis may be transient or stable transfection of a cell or a cell line, an episomal expression system in a cell or a cell line, or preparation of a transgenic non-human animal by pronuclear microinjection, through recombination events in embryonic stem (ES) cells or by transfection of a cell whose nucleus is to be used as a donor nucleus in a nuclear transfer cloning procedure.

In embodiments of the invention relating to the preparation of a transfected host cell or a transgenic non-human animal comprising the use of a nucleic acid construct as previously described, the cell or non-human animal may be one that has undergone previous genetic modification resulting in the selective deletion or inhibition of expression of one or more endogenous genes. The said gene deletions or inhibitions may be effected by permanent deletion of selected sequences from the genome, conditional deletion of selected sequences, for instance by insertion of *lox*P sequences flanking the sequences to be deleted and subsequent conditional excision of said sequences to be deleted by constitutive or conditional expression of cre recombinase (US4959317) or selective interference with RNA transcripts, for instance by insertion into the genome of a DNA sequence directing expression of a short double-stranded RNA molecule with one strand complementary to the target gene (US6,573,099).

Introduction of the nucleic acid construct as previously described into a cell or non-human animal in which genetic modification resulting in the selective deletion or inhibition of expression of one or more endogenous genes has been effected, may be achieved by transient or stable transfection of a cell or a cell line in which deletion or inhibition of another gene has previously been effected, or by introduction of an episomal expression system into a cell or a cell line in which deletion or inhibition of another gene has previously been effected, or by preparation of a transgenic non-human animal by pronuclear microinjection, through recombination events in embryonic stem (ES) cells from an animal in which deletion or inhibition of another gene has previously been effected or by transfection of a cell from an animal in which deletion or inhibition of another gene has previously been effected whose nucleus is to be used as a donor nucleus in a nuclear transfer cloning procedure or by cross-breeding an animal as described under the fourth aspect of the present invention with another animal of the same species in which deletion or inhibition of another gene has previously been effected and subsequent genomic analysis of offspring of the crossing to identify individuals in which both the introduced nucleic acid construct of the present invention and the deletion or inhibition of the other selected gene are present, for instance by isolation of genomic DNA from a tissue sample and PCR analysis.

Methods of preparing a transgenic cell or cell line, or a transgenic non human animal, in which the method comprises transient or stable transfection of a cell or a cell line, expression of an episomal expression system in a cell or cell line, or pronuclear microinjection, recombination events in ES cells, or other cell line or by transfection of a cell line which may be differentiated down different developmental pathways and whose nucleus is to be used as the donor for nuclear transfer, wherein expression of an additional nucleic acid sequence or construct is used to screen for transfection or transgenesis in accordance with previous aspects of the invention. Examples include use of selectable markers conferring resistance to antibiotics added to the growth medium of cells. For instance neomycin resistance marker conferring resistance to G418. Further examples involve detection using nucleic acid sequences that are of complementary sequence and which will hybridise with, or a component of, the nucleic acid sequence in accordance with the first, second, third, or fourth aspects of the invention. Examples would include Southern blot analysis, northern blot analysis and PCR.

According to another aspect of the invention, there is provided the use of a nucleic acid construct in accordance with any one of the previous aspects of the invention for the detection of a gene activation event resulting from a a toxic insult in a cell *in vitro.*

The gene activation event may be the result of induction of toxicological stress.

According to another aspect of the invention there is provided the use of a nucleic acid construct comprising a Reporter Sequence, as defined under the first aspect of the present invention, wherein the transcription and/or translation products of said Reporter Sequence are heterologous to the cell in which the Reporter Sequence is expressed, for the detection of a gene activation event resulting from a toxic insult in a cell *in vitro.*

The gene activation event may be the result of induction of toxicological stress.

Uses in accordance with previous aspects of the invention also extend to the detection of disease states or characterisation of disease models in a cell, cell line or non human transgenic animal where a change in the gene expression profile within a target cell or tissue type is altered as a consequence of the disease. Diseases in the context of this aspect of the invention which are detectable under the methods disclosed may be defined as infectious disease, cancer, inflammatory disease, cardiovascular disease, metabolic disease, neurological disease and disease with a genetic basis.

An additional use disclosed involves the growth of a transfected cell line in accordance with the third aspect in a suitable immunocompromised mouse strain (referred to as a xenograft), for example, the nude mouse, wherein an alteration in the expression of the reporter described in the first or second aspects of the invention may be used as a measure of altered metabolic status of the host as a result of toxicological stress, metabolic changes, disease with a genetic basis or disease that may or may not be the result of viral, bacterial, fungal or parasitic infection. The scope of this use may also be of use in monitoring the effects of exogenous chemicals or drugs on the expression of the reporter construct.

The previous aspects of the invention extend to methods of detecting a gene activation event *in vitro* or *in vivo.*

In an embodiment according a previous aspect of the invention, the method comprises assaying a host cell stably transfected with a nucleic acid construct in accordance with any one of the first or second aspects of the invention, or a transgenic non-human animal according to the fourth aspect of the invention, in which the cell or animal is subjected to a gene activation event that is signalled by expression of a Reporter Sequence whose translation product is identified by means of an epitope tag peptide sequence.

In an embodiment according to a previous aspect of the invention, the method comprises assaying a host cell stably transfected with a nucleic acid construct comprising a Reporter Sequence, wherein the transcription or translation products of said Reporter Sequence is heterologous to the cell in which it is expressed, or a transgenic non-human animal whose cells express such a construct, in which the cell or animal is subjected to a gene activation event that is signalled by expression of a Reporter Sequence whose translation product is identified by means of an epitope tag peptide sequence.

According to a yet further aspect of the invention there is provided a method of screening for, or monitoring of toxicologically induced stress involving Hmax-1 gene activation in a hepatic or renal cell or a cell line or a non-human animal, comprising the use of a cell, cell line or non human animal which has been transfected with or carries a nucleic acid construct as described above.

Preferably, the cell or a cell line or a non-human animal may comprise more than one gene construct according to the present invention in order that a variety of stress conditions may be monitored or screened simultaneously in a single cell or a cell line or a non-human animal. It will be appreciated that in this way a number of different read-outs may be obtained.

Alternatively in the method of the present invention a set or number of renal and/or hepatic cells, cell lines or non human animals may be used simultaneously each comprising different gene construct(s ) according to the present invention so that a variety of stress conditions may be screened or monitored simultaneously in a set or group. It will be appreciated that in this way a number of different read-outs may be obtained.

Toxicological stress may be defined as DNA damage, oxidative stress, post translational chemical modification of cellular proteins, chemical modification of cellular nucleic acids, apoptosis, cell cycle arrest, hyperplasia, immunological changes, effects consequent to changes in hormone levels or chemical modification of hormones, or other factors which could lead to cell damage.

Accordingly, there is additionally disclosed a method for screening for cancer, inflammatory disease, cardiovascular disease, metabolic disease, neurological disease and disease with a genetic basis comprising the use of a cell, cell line or non human animal which has been transfected with or carries a nucleic acid construct as described above.

In these contexts the cell may be transiently transfected, maintaining the nucleic acid construct as described above episomally and temporarily. Alternatively cells are stably transfected whereby the nucleic acid construct is permanently and stably integrated into the transfected cells' chromosomal DNA.

Also in this context transgenic animal is defined as a non human transgenic animal with the nucleic acid construct as defined above preferably integrated into its genomic DNA in all or some of its cells.

Expression of the Reporter Sequence whose translation product is identified by means of an epitope tag peptide sequence in respect of the fifth aspect of the invention can be assayed for by measuring levels of the Reporter Sequence translation product in cell culture medium or purified or partially purified fractions thereof.

Skilled persons will appreciate how to choose Reporter Sequences whose translations products are known to be secreted into body fluids. For instance, a Reporter Sequence could be chosen to encode a lipocalin or the beta chain of human choriogonadotrophin, or secreted alkaline phosphatase any of which are secreted from the cells in which they are expressed and are eliminated into urine. Expression of a Reporter Sequence in accordance with the fourth aspect of the invention therefore can be assayed for by measuring levels of the translation product of said Reporter Sequence secreted into harvestable body fluids. In a preferred embodiment of the invention the body fluid will be urine, but may also be selected from the list including milk, saliva, tears, semen, blood and cerebrospinal fluid, or purified or partially purified fractions thereof.

Detection and quantification of Reporter Sequence translation products from cultured cells into tissue culture medium or transgenic non-human animal body fluid may be achieved using a number of methods known to those skilled in the art:
1. Immunological methods.
   (i) The assay may be an ELISA whereby an antibody or antiserum containing a single or mixture of antibodies recognising translation product of the Reporter Sequence and is used as a capture antibody to coat a microtitre plate or other medium suitable for conducting the assay. The culture medium or body fluid containing the reporter gene product (analyte) is added to the microtitre plate to allow binding of the analyte. Addition of the same antibody or antiserum that has been conjugated to an enzyme, commonly horseradish peroxidase, is used as a second antibody. Addition of a suitable substrate, preferably one producing a colour product following conversion by the enzyme is used to quantify the analyte in proportion to how much second antibody conjugate has been bound.
   (ii) Competitive ELISA. In an alternative form the tissue culture medium or the body fluid (analyte) sample containing the expressed Reporter Sequence translation product bound to a support suitable for conducting the assay. In a separate reaction a limited standard amount of antibody specifically recognising the reporter gene product is added to a separate aliquot of the same and allowed to bind. This is added to the analyte bound to the support to allow remaining free antibody to bind. A second, enzyme conjugated antibody against for example the Fc region of the first antibody is allowed to bind and the colorimetric readout can be used to quantify the analyte whereby the degree of colour change is inversely proportional to the level of analyte in the sample.
   (iii) Western blot analysis
      Transfected cell homogenates were prepared by incubation of cells in homogenization buffer (140mM NaCl, 50mM Tris-HCl pH7.5, 1mM EDTA, 1% Triton-100) for 30 minutes on ice. Following a brief centrifugation to remove insoluble material the cleared supernatants were assayed for protein content. A volume equivalent to 40µg cell extract and an equal volume of cell medium were subjected to SDS-PAGE and blotted onto nitrocellulose (Schleicher and Schuell, Dassel, Germany) membrane using a semi-dry blotting apparatus (Bio-Rad, Richmond, CA). The membranes were blocked for 1 hour in blocking buffer (5% NFDM w/v in PBS) then incubated with myc mAb (Invitrogen Life Technologies, Carlsbad, CA) diluted in blocking buffer for 2 hours with continuous agitation. After a series of washes in PBST (PBS plus 0.05% Tween-20), the membrane was incubated in an anti-mouse antibody conjugated to HRP diluted in blocking buffer for one hour with agitation, and after another series of washes in PBST the HRP activity was developed using an ECL kit (Pierce, Rockford, IL) and captured on auto-radiographic film (Kodak).
   (iv) Fluorescence polarisation. The antibody specifically recognising the Reporter Sequence translation product is conjugated with fluorescein and mixed with the analyte produced. This method quantifies the analyte by direct measurement of the amount of antibody-antigen complex present. This method may also be adapted to measure any protein-protein interaction.
2. Release of a labelled substrate. Detection of conversion of substrate due to enzymatic activity of the translation product of a Reporter Sequence. The nature of substrate conversion may or may not fall into one or more of the following event categories: Proteolysis, phosphorylation, acetylation or sulphation, methylation
3. Detection of multiple substrates. Where a multiplicity of Reporter Sequence translation products are used, methods suitable for detection of such events could include but not necessarily be limited to:
   (i) Mass spectrometry
   (ii) Nuclear magnetic resonance (NMR)

In a preferred embodiment of the invention there is provided a method of detecting a reporter gene activation event, comprising the steps of:
1. Transfecting a cell or microinjecting the pro-nucleus of a fertilised mouse egg with a nucleic acid construct in accordance with the first, second, third, or fourth aspects of the invention. Optionally use the microinjected egg or transfected mouse ES cell line;
2. Exposing the transfected cell, cell line or transgenic non human animal to a stimulus which may or may not cause a change in metabolic status resulting alteration in gene expression; and.
3. Using a suitable assay to determine the level expression of the Reporter Sequence translation product, for example using detection methods such as ELISA, RIA, Mass spectrometry, NMR, telemetric methods.

In step (1), the Reporter Sequence transcription and/or translation products may be heterologous to those already expressed in the cell in which the Reporter Sequence is expressed. In any case, the skilled person will appreciate how to engineer a Reporter Sequence with tagging sequences so that transcription and/or translation products of said Reporter Sequence will be heterologous to those already expressed in the cell in which the Reporter Sequence is expressed.

Methods and uses in accordance with the present invention offer significant advances in investigating any area in which modified gene expression plays a significant role. Such reporter genes will be of use in cells and transgenic animals to detect activity of the p21 gene. Specific applications include but are not restricted to:
1. Providing a rapid and robust in vivo screening system for assessing the potential toxic effects of chemicals.
2. Provide information on the mechanism of toxicity. Such information could be used to eliminate compounds from a selection process or suggest possible modifications to a compound.
3. Provide information on the effect of combinations of compounds.
4. Allow monitoring of variation In reporter gene expression over time by measuring levels of reporter(s) in urine at different time intervals.
5. Assessment of changes in gene expression associated with pathogenic infection.
6. Assessment of changes in gene expression associated with neurological, cardiovascular and metabolic diseases.
7. Assessment of changes in gene expression associated with cancer.
8. Provide information allowing validation of drug target selection for instance by matching reporter expression profile to actions of toxins whose mechanism is defined and understood.
9. Use for evaluating compounds as therapeutic strategies aimed at reversing a toxic, metabolic, or degenerative phenotype.
10. Assessment of changes in gene expression resulting from environmental and/or behavioural changes.

Preferred features for the previous aspects of the invention are as for the first aspect *mutatis mutandis.*

The present invention will now be described with reference to the following examples which are present for the purposes of illustration only and should no be construed as being limited with respect to the invention. Reference in the application is also made to a number of drawings in which:

### BRIEF DESCRIPTION OF THE DRAWINGS

Figure 1 part (a) shows the sequences of PCR primer oligonucleotide pairs used to amplify four regions of the mouse p21 gene and its promoter and part (b) shows reconstruction form these four amplified PCR products of a 5006 base pair DNA construct comprising Promoter Sequence, Transcription Start Sequence and noncoding exon suitable for combination with a Reporter Sequence to make a complete reporter construct.
Figure 2 shows the pX3W construct.
Figure 3 shows the pXWhCG.RAB construct.
Figure 4 shows the expression of LacZ in acrylamide-treated WAZ44 mouse brain.
Figure 5 shows the expression of LacZ in WAZ44 mouse brain
Figure 6 shows the histopathology of HOZ mouse liver and kidney following treatment with cadmium chloride.
Figure 7 shows the histology of HOZ mouse liver with and without cadmium chloride treatment.
Figure 8 shows the effect of cadmium chloride on HOZ transgene expression in liver 24 hours after administration.
Figure 9 shows the effect of cadmium chloride on HOZ transgene expression in liver 8 hours after administration.
Figure 10 shows the histology of HOZ mouse kidney with and without cadmium chloride treatment.
Figure 11 shows the induction of HO-1 mRNA ion naïve, vehicle control and cadmium chloride-treated HOZ mice.
Figure 12 shows HO-1 mRNA levels in HOZ mouse liver 8 hours and 24 hours after administration of various does of cadmium chloride.
Figure 13 shows sodium arsenite induces HOZ transgene expression in mouse liver.

### Material and Methods

### Generation of p21 reporter constructs

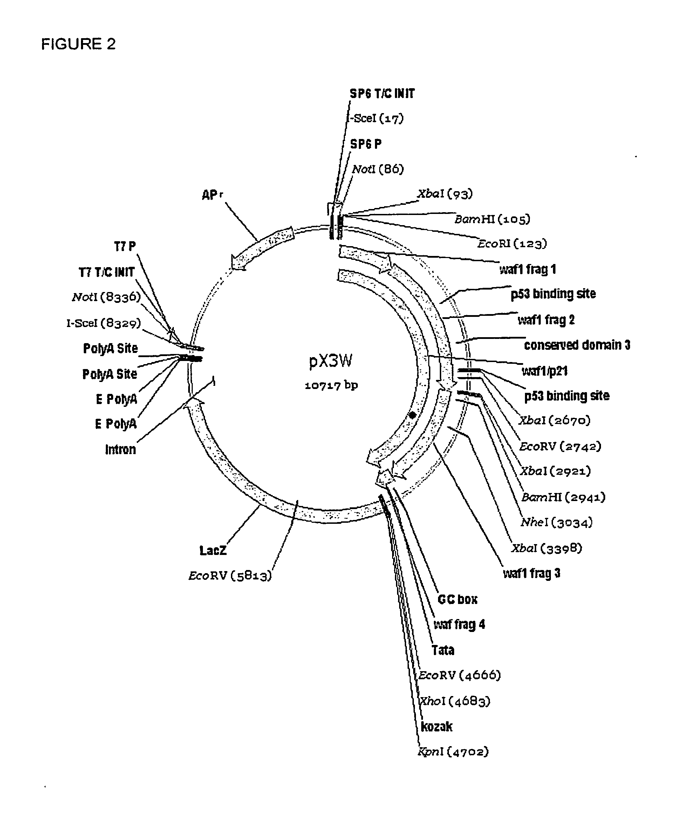

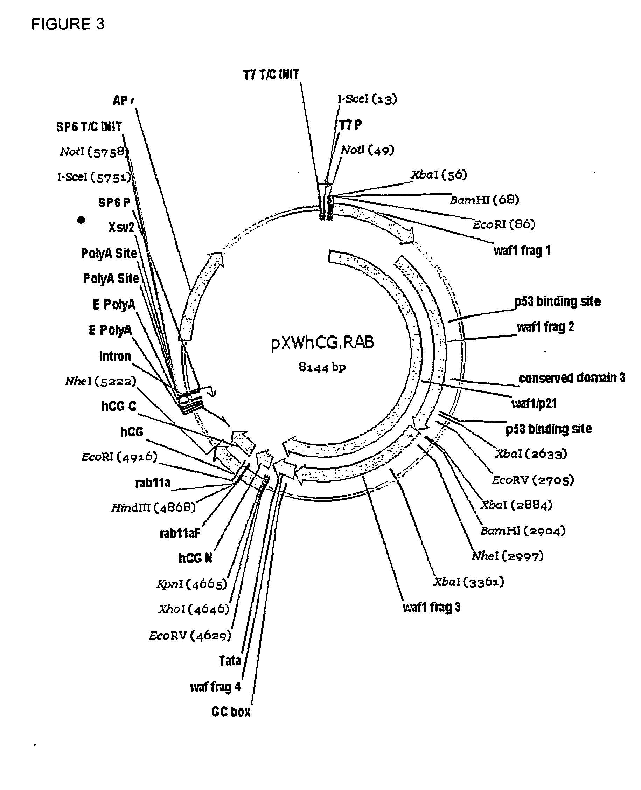

Two reporter constructs have been generated. Both contain a 4.5kb fragment encompassing a contiguous segment of the mouse p21 gene promoter and upstream transcriptional control sequences. In one the p21 promoter segment is upstream from the coding sequence for a LacZ reporter gene and in the other it is upstream from the coding sequence for human chorionic gonadotrophin tagged with a unique epitope designed to facilitate detection by immunological assays. These constructs are referred to as pX3W (Figure 2) and pXWhCG.RAB (Figure 3) respectively. Maps of these are shown in Figures 2 and 3.

### Generation of WAZ transgenic mice

The p21-LacZ and/or the p21-hCG construct is microinjected into fertilised mouse eggs which are implanted into pseudo-pregnant surrogate mothers. Pups are screened for the presence of the LacZ reporter gene by PCR analysis of tail biopsy tissue lysates. The sequence of the primers used for PCR screening are:
Forward primer 5'-GAC ACC AGA CCA ACT GGT AAT-3', (SEQ ID NO:1)
Reverse primer 5'-GCA TCG AGC GTA ATA AGC-3'. (SEQ ID NO:2)

Samples are cycled as follows: 5.00 min @ 94°C, 35 cycles of (0.30 min @94 °C, 0.30 min @ 61 °C, 2.00 min @72 °C) 7.00 min @ 72 °C, α @ 4°C.

Transgenic individuals identified by this method are bred with non transgenic animals to determine germline transmission of the transgene and establish transgenic mouse lines for analysis. All mice are maintained in a pathogen free environment.

### Line Selection

Initial screening of transgenic offspring from founders generated and identified as described, is conducted by IP injection of a suitable compound known to induce activity of the wild type p21 gene; For example the topoisomerase II inhibitor etoposide is known to result in DNA damage. If dissolved in DMSO and injected intra-peritoneally at 40mg/kg (LD₅₀), subsequent examination of tissue sample sections by staining to detect LacZ reporter gene expression may be used to examine reporter gene expression characteristics of individual transgenic lines.

### Targeted Transgenesis

Targeted insertion of transgenes at the hypoxanthine phosphoribosyltransferase (Hprt) locus, on the mouse X chromosome was performed by Nucleis S.A., 60 Avenue Rockefeller, 69008 Lyon, France. The Hprt gene is a housekeeping gene, and as such is ubiquitously expressed in all tissues. This locus has been shown to exhibit no intrinsic enhancer activity in the majority of tissues and provides an exquisitely permissive chromatin environment that supports promoter dependent transgene expression (Farhadi et al., 2003; Heaney et al., 2004). Targeted insertion is achieved using BPES embryonic stem cells, in which the murine Hprt locus has been partially deleted, in combination with an Hprt targeting vector, pDEST, designed to restore Hprt gene function, and into which the transgene is cloned. The transgene is introduced into the pDEST targeting vector (Gateway® cloning, Invitrogen) by means of lambda phage-based site-specific recombination. In order to utilize this system, it is first necessary to create a low copy number plasmid that contains specific recombination sites (attL), a so-called Entry vector. This can be used initially to construct, then later to shuttle the transgene to the pDEST destination vector by recombination. The vector is generated by introducing the specific recombination sequences attL1 and attL2, separated by a spacer fragment, into the low copy number plasmid pACYC177, to create plasmid pLC15att. This vector is used for the subsequent cloning of reporter constructs.

### Animal husbandry

Mice were housed on sawdust in solid-bottom, polypropylene cages and acclimatised for a period of 5 days before use. Temperature was maintained within a range of 19 - 23°C and relative humidity within a range of 40 - 70%. There were 14 -15 air changes per hour and twelve-hour periods of light were cycled with twelve-hour periods of darkness. Animals received RM1 diet (supplied by Special Diet Services Ltd., Stepfield, Witham, Essex, UK) *ad libitum* for the duration of each study. Drinking water, taken from the local supply and provided in bottles, was provided *ad libitum.* Animals were randomly allocated to groups, ear-numbered and weighed prior to the start of each experiment.

### Dose preparation and administration

Each test compound was dissolved in the appropriate vehicle solution and administered by intraperitoneal injection (i.p.) at a dose volume of 10ml/kg. Wherever possible, both naive (untreated) and vehicle-treated controls were used. If a test item whose toxicity in the mouse is poorly characterised was to be used, a dose-ranging experiment was conducted prior to the main experiment. The test item was administered to a single mouse which was monitored for any adverse effects 6-8 hours and 24 hours later. If the single dose ranging mouse tolerated the treatment, the remaining mice were administered the test item according to the protocol.

### Termination procedures

Terminal blood samples were taken by cardiac puncture into lithium/heparin-coated tubes for preparation of plasma. Tissues, as defined in each protocol, were routinely dissected as follows:
Two slices of tissue (∼ 2 mm thickness) were removed for histochemical analysis of LacZ expression according to methods described below.
Two further slices (∼ 2 mm thickness) were placed in 10% neutral buffered formalin (10% NBF) overnight then processed, sectioned and stained with haematoxylin and eosin (H&E).

The remaining tissue was placed in a cryovial and flash frozen in liquid nitrogen for RT-PCR and possible biochemical analysis.

Mouse brains were dissected so as to reveal the dentate gyrus as fully as possible. In the case of the kidneys, one kidney was cut in longitudinal section and the other in transverse section.

### Analysis of plasma samples

Following removal into tubes suitable for plasma preparation, venous blood samples were mixed on a roller for 10 minutes then cooled on ice. Red blood cells were removed by centrifugation at 2,000rpm for 10 minutes at 8 - 10°C. The supernatant (plasma) was transferred to a second tube and stored at -70°C until required for analysis.

Plasma samples were analysed for appropriate analytes on a Roche autoanalyser (Cobas Integra 400) according to the manufacturer's instructions. The routine markers measured included: (for liver damage) plasma alanine aminotransferase (ALT), aspartate aminotransferase (AST) and alkaline phosphatase (ALP) and (for kidney damage) plasma creatinine and blood urea nitrogen (BUN).

### Reverse Transcription-Polymerase Chain Reaction (RT-PCR) Assay of mRNA

Analysis of the expression of endogenous genes was carried out by RT-PCR using the ABI Prism 7000 system. For RT-PCR analysis, frozen tissue samples were homogenised using a Polytron PT-mr2100 homogeniser in 1ml TRI reagent (Sigma). Total RNA for each sample was isolated according to the manufacturer's instructions and RNA pellets stored at -70°C under 75% ethanol. Each of the RNA pellets was resuspended in RNAase/DNAase free dH₂O and quantified using a Genequant RNA/DNA calculator (Amersham). First strand cDNA synthesis was performed for each sample using 2µg of total RNA and the Protoscript First Strand cDNA Synthesis Kit (New England Biolabs) according to the manufacturer's protocol. Additional steps to degrade RNA following cDNA synthesis were performed. Each sample was diluted to 50µl with RNAase/DNAase-free dH₂O and stored at -20°C.

In order to measure variation in p21 or Haemoxygenase-I (HO-1) expression it was necessary to include an internal control. Amplification of β-Actin cDNA provides a standard to control for variations on RNA content between samples. The final reactions therefore consisted of two separate reactions (target gene and β-Actin cDNA amplification) performed simultaneously in a single tube. The PCR conditions used were 10 minutes at 95°C followed by 40 cycles of denaturing for 15 seconds at 95°C and annealing/extending for 1 minute at 60°C. The primers and probe for the p21 and HO-1 amplification reactions were obtained from ABI (Assay-On-Demand kit). These methods have been pre-validated for the standard conditions used on the ABI Prism 7000 system.

### Detection of LacZ transgene expression

### • Frozen Method 1

Tissues were washed with PBS and prefixed in 0.2% glutaraldehyde in PBS containing 0.1 M MgCl₂ and 5mM EDTA, pH 7.3, for 4 hours at 4°C on a shaker. After fixation, the tissues were transferred to 20% Sucrose solution and dehydrated overnight at 4°C. The next day they were embedded in cryo-M-bed medium on cork discs and lowered slowly into liquid nitrogen then transferred to -80°C until sections were required.

Cryostat sample and chamber temperatures were set to -30°C. The sample to be sectioned was placed in the cryostat one hour prior to sectioning to allow equilibration at the appropriate temperature. Frozen sections (10µm) were cut and placed on aminopropyltriethoxysilane (APES) coated slides.

Prior to staining, sections were incubated in 0.2% glutaraldehyde (as above) for 10 minutes at room temperature then washed twice (5 min each) in PBS containing 2mM MgCl₂, 0.01% sodium deoxycholate and 0.02% % Nonidet-P40. Sections were then more of the same wash solution containing 1 mg/ml X-gal, 5 mM potassium ferrocyanide and 5 mM potassium ferricyanide overnight at 37°C then washed twice in PBS for 5 min, allowed to air dry and mounted in aqueous mounting medium (Hydromount). Slides were evaluated and photographed.

### • Frozen Method 2

This method was as described by Campbell et al., 1996 and Campbell et al., 2005, with minor modifications.

Tissues were excised, immediately placed into chilled 4% paraformaldehyde containing 0.1% glutaraldehyde and 2mM MgCl₂ in PBS, and fixed in the above solution for 3 hours at 2 - 8°C with gentle shaking. Fixed tissues were washed at 2 - 8°C for 3 x 5minutes with chilled PBS containing 2mM MgCl₂ and dehydrated overnight at 2 - 8°C in PBS containing 2mM MgCl₂ and 30% (w/v) sucrose overnight The next day dehydrated tissues were embedded in OCT embedding medium using an isopentane/dry ice bath. Embedded tissues were stored at -70°C until they could be cryosectioned.

Frozen sections were washed three times for 5 minutes in PBS containing 2mM MgCl₂ at 2 - 8°C then 3 x 30 min in detergent wash (PBS containing 2mM MgCl₂. 0.01% Nonidet P40 and 0.1% Sodium Deoxycholate) at 2 - 8°C. They were then stained overnight in PBS containing 2mM MgCl₂, 0.02% Nonidet P40, 0.1% Sodium Deoxycholate, 5mM Potassium ferrocyanide, 5mM Potassium ferricyanide, and 0.1% X-gal at 37°C. The next day, the slides were washed three times for 5 minutes in PBS containing 2mM MgCl₂ followed by three times for 5 minutes in PBS at room temperature. They were equilibrated with 100% ethanol (5 min) and counterstained with Eosin for 1 min at room temperature. Following dehydration and mounting they were evaluated and photographed.

### • Whole Mount Method

Tissue specimens that had been prefixed in fresh 4% paraformaldehyde were stained immediately using the procedure for "Whole Mount X-Gal Histochemistry of Transgenic Animal Tissues" (see http://www.rodentia.com/wmc/docs/lacZ bible. html), summarised below) was followed, and the post-fixed tissues were transferred to 70% (v/v) ethanol.

Following fixation for one hour in 4% paraformaldehyde, tissues were rinsed three times, for thirty minutes each, with rinse buffer (100 mM sodium phosphate (pH 7.3), 2 mM MgCl2, 0.01% sodium deoxycholate, 0.02% NP-40) at room temperature. They were then stained in rinse buffer plus 5 mM potassium ferricyanide, 5 mM potassium ferrocyanide, 1 mg/ml X-gal (made up from 25 mg/ml stock in dimethylformamide (DMF), stored at -20°C) for between 4 and 48 hours (typically overnight; about 90% of potential staining is believed to occur in the first 24 hours). Subsequently, the tissues were post-fixed overnight in 10% formalin at 4°C and processed, sectioned and counterstaining with Neutral Red to reveal the nuclei.

### EXAMPLES

### Example 1; p21 Reporter Gene Construct

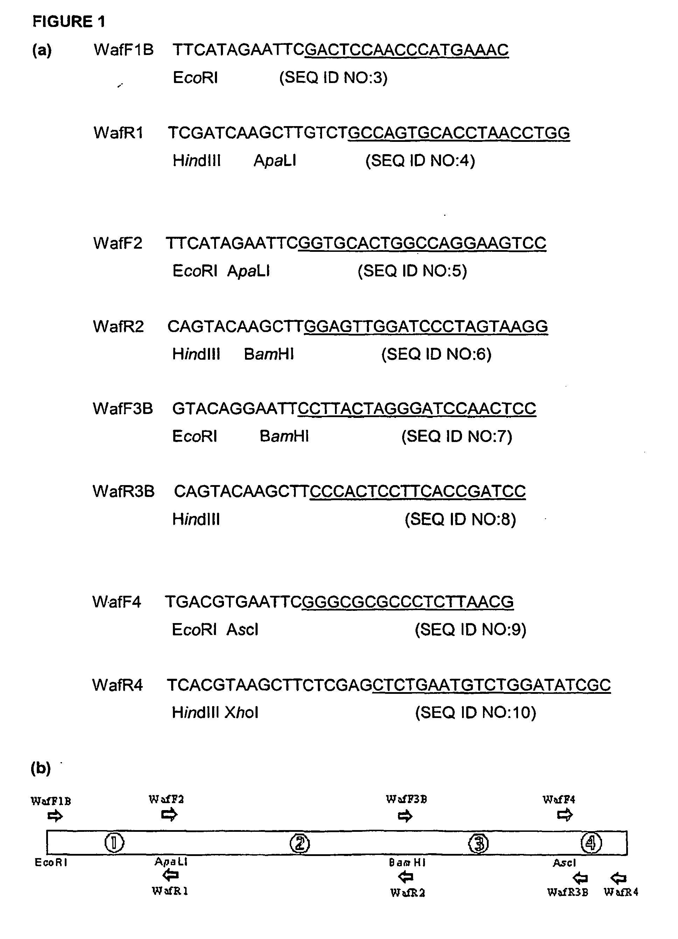

Four sections of the promoter region of p21 (el-Deiry, W.S. et al., Cancer Res. 55: 2910-2919, 1995; Ensembl Mus-musculus transcript information ENSMUST00000023829) were individually amplified by polymerase chain reaction (PCR) from mouse genomic DNA (CB/4) using Herculase Taq (Stratagene) using the oligonucleotide primer pairs shown in Figure 1(a). Primer sequence sections complementary to sequences from the mouse p21 promoter region are shown underlined. Each PCR product was separately digested with H*in*dIII and E*co*RI and ligated into pBuescript. Following sequence validation, the four fragments were ligated into a single construct using the unique internal A*pa*LI, B*am*HI and Ascl restriction sites and the engineered Ec*o*RI and X*ho*I flanking sites to produce a 5006 base pair construct comprising the first 87 base pair noncoding exon of p21 and sequences upstream from it (Figure 1b). The complete p21 promoter construct was then excised with E*co*RI and X*ho*I and ligated into vector pXen3 upstream of the Kozak sequence, *LacZ* coding sequence and SV40 polyadenylation signal sequence to yield vector pX3W (Figure 2). Transgenic WAZ mice were produced by microinjection of pX3W into pronuclear C57BL/6xCBA embryos.

### Example 2; Metallothionein 1A Reporter Gene Construct

Also referred to as mt1, one of a family of small cysteine rich proteins with potent metal binding and redox capabilities, Mt1 plays an important role in Zinc homeostasis, and cellular response to heavy metal toxicity and oxidative damage. A complex array of transcription factor binding sites have been identified in the proximal promoter from -700bp to the transcription start site. These include five MREs (MTF-1 binding), Sp1, ARE, USF1, AP-1 ( plus AP-2, AP-4 in human). The five MREs clustered in the proximal promoter region (-153 to -43) participate in mediating transcriptional induction in response to heavy metals and to oxidative stress (Stuart et al., 1984 Proc Natl Acad Sci U S A. 1984 Dec;81(23):7318-22, Dalton et al., 1996 J Biol Chem.;271(42):26233-41). An overlapping ARE/USF recognition sequence at -100 is essential in maintaining basal expression and also plays a role in the activation of Mt1 expression in response to oxidative stress. MTF-1 and USF-1 have been reported to cooperate in essential regulation of Mt-1 expression in response to zinc in the developing embryo (Andrews et al., 2001 EMBO J 20: 1114-112). The ARE mediates induction of GST-Ya subunit and the quinine reductase genes in response to H₂O₂ and redox cycling xenobiotics (Jaiswal, 1994 Biochem Pharmacol. 3;48(3):439-44.). Reporters already constructed (LacZ and tagged hCG) for the mouse gene incorporate a 26kb upstream segment of the gene, designed to encompass as yet uncharacterized upstream transcriptional modulatory elements. Others have made and studied mouse knockout models for mt1 and mt2: Mice homozygous for disruption of both alleges developed normally but were markedly more susceptible to hepatic poisoning with Cd than wild type (Masters et al., 1994 Proc. Nat. Acad. Sci. 91: 584-588). Beattie et al. (1998, Proc. Nat. Acad. Sci. 95: 358-363, 1998) also reported increased susceptibility to toxic stress, higher food intake and resultant obesity in mt1/ mt2 double null mice. Amongst the stimuli eliciting elevated transcription of mt1 are heavy metals (e.g. cadmium) (Li et al., 1998 Nucl. Acids. Res. 26: 5182-5189), cobalt, zinc, H₂O₂, hydroxyl radical, heme analogues, ischemia, carbon tetrachloride, tin protoporphyrin, glucocorticoids, Phorbol esters, paraquat, diethyl and pyrrolidine dithiocarbamate, LPS, X-ray irradiation and inflammatory stress signals.

### Example 3; PUMA Reporter Gene Construct

p53 up-regulated modulator of apoptosis, also referred to as BBC3. Exogenous expression of PUMA has been shown to result in extremely rapid apoptosis in cells in which it is expressed (Yu. J *et al* 2001). Its transcription, at least in part is elevated by binding of p53 to cognate promoter binding sites within the proximal promoter (p53 binding site BS1 at 230bp upstream of transcriptional start and BS2 at 144bp upstream of transcriptional start) are major p53 response elements characterized in the human promoter. Counterparts in the human promoter are sited at -410bp and -375bp respectively from the transcription start. However as the promoter region of PUMA is not well characterized reporters incorporate up to 16kb of upstream gene sequence and 5kb of downstream sequence so that as yet uncharacterized transcriptional control elements are included.

It is therefore envisaged that a PUMA gene reporter will be useful for detecting toxic insults that elicit apoptosis in a cell. Known inducers include DNA damaging agents such as 5FU, Adriamycin, Etoposide (Yu. J et al Proc. Nat. Acad. Sci. 100: 1931-1936, 2003, Yu. J et al Molec. Cell 7: 673-682, 2001, Nakano, K Molec. Cell 7: 683-694, 2001) and agents known to increase cellular level of p53 such as 4-hydroxytamoxifen (4-OHT) (Jia-wen Han et al 2001 PNAS 98 (20) 11318-11323).

### Example 4; Gclc Reporter Gene Construct

Glutamate-cysteine ligase catalytic subunit (GCLC) along with the modulatory unit (GCLM) catalyses the first step in glutathione (GSH) biosynthesis which plays an essential role in protection of the cell against a range of toxic insults that cause accumulation of free radicals. Gclc transcription is know to be elevated by a range of chemical/ xenobiotic agents, E.g. Chloroform, Cadmium Chloride, Acetaldehyde (Yang H. et al 2001 Biochem. J. (2001) 357: 447-455), Pyrrolidine dithiocarbamate (PDTC) in HepG2 and HEK293 cells (Erica L. Dahl et al 2001 Toxicological; Sciences 61, 265-272), Phenethyl isothiocyanate(PEITC) in HepG2 and HEK293 cells (Erica L. Dahl et al 2001 Toxicological; Sciences 61, 265-272), β-napthoflavone (β-NF) in HepG2 cells (Erica L. Dahl et al 2001) and cadmium chloride. A number of regions of the promoter are know to modulate gclc gene transcription; E.g. Yang *et al* (2001) conducted functional analysis of the promoter and describe three upstream flanking regions located between -595 to -111, -1108 to -705 (conferring positive positive regulation) and one between -705 to -595 (conferring negative regulation)

Within the proximal promoter at -416 to -316 NF-κB and AP-1 recognition sequences exist. Analysis of the human 5' flanking region show it to contain an antioxidant-response element (ARE) and AP-1 and NF-κB binding sites. Knockout of the mouse glutamate cysteine ligase catalytic subunit (Gclc) gene is embryonic lethal when homozygous, and has been proposed as a model model for moderate glutathione deficiency when heterozygous (Dalton et al., 2000 Biochem Biophys Res Commun. Dec 20;279(2):324-9). It is therefore envisaged that reporters based on gclc will provide a reliable indicator of whether the test system (cells or transgenic animal) is being placed under oxidative stress. Reporters are designed such that coding region of the reporter gene is placed in the second exon, at the position of the natural first codon of the gclc locus, with 163kb of upstream sequence providing known and putative uncharacterized transcription regulatory elements.

### Example 5; Cox2 Reporter Gene Construct

Murine Cyclooxygenase-2, also referred to as prostaglandin-endoperoxidase synthase 2 (Ptgs2) is responsible for catalysing the conversion of arachidonic acid to prostaglandins. It is induced in response to inflammatory signals e.g. IL-6 and is the main target for non-steroidal anti-inflammatory drugs such as aspirin. The literature reports a wide range of compounds that result in elevated expression of cox-2, E.g. Lipopolysaccharide, Cytokines, Mitogens: Superoxide, hydroxy radical,Arsenite, CCl₄, 4-Hydroxy-2-nonenal, LPS, interleukin-1, tumor necrosis factor-alpha, PAF or interleukin-1beta, benzo[a]pyrene, ethanol and arachidonic acid, Phorbol 12-myristate 13-acetate, doxorubicin, Fe-NTA, deoxycholic acid12-O-tetradecanoylphorbol 13-acetate.

An expression cassette based on Cox2 potentially represents a good candidate reporter for any toxic insult eliciting an inflammatory response. The promoter region of the gene contains a complex array of known transcription factor binding sites such as NF-kB, (NF-kappaB-2 but not NF-kappaB-1), NF-IL6, ATF/CRE, E-Box, Pea3, SP1, AP-2, C/EBP, STAT3, STAT1,TBP-response elements. The highly conserved 3'UTR is implicated in post-transcriptional control mediated at the level of mRNA stability, E.g. Phosphatidyl-inositol 3-kinase and AgC10 negatively regulate Cox-2 mRNA via the 3'UTR by inhibiting p38 MAPK required for the stability of the message. The reporter constructs therefore incorporate a ∼3.5kb segment upstream from the transcription start site and a further -3kb segment downstream from the coding sequences of cox2.

### Example 6; Ki67 Reporter Gene Construct

The name refers to the commercially available antibody recognizing a very large nuclear antigen highly expressed in proliferating cells (Schluter, C et al J. Cell Biol (1993) 123: 513-522). Expression of the antigen occurs during early G1, S, G2 and M phase of the cell cycle but is not detected in quiescent cells. Though the antibody is used widely in tumour pathology to detect proliferating cells in neoplastic disease states little is know about its function, though clearly its ubiquity in dividing cell populations suggests a cental role in cell division. The absolute correlation between high levels of Ki67 expression and cycling cell populations make it a potentially powerful tool on which to base a reporter gene for cell proliferation. As the gene and its regulation are as yet poorly characterized reporter gene design will utilize a large upstream segment of the gene (up to 30kb upstream of the transcription start) that is designed to encompass as yet unidentified regulatory elements.

### Example 7; Stk6 Reporter Gene Construct

Serine/Threonine Kinase 6 (Aurora-family kinase 1), STK6, controls various mitotic events. The transcription of STK6 gene varies throughout the cell cycle and peaks during G2/M. The promoter of mouse STK6 has been characterized. An E4TF1 motif (-87 to -78) and an Sp-1 site (-129 to -121) have been identified as positive regulatory elements (Tanaka M et al (2002) JBC Vol. 277:10719-10726). A tandem repressor element, CDE/CHR(-53 to -49/-39 to -35), has been identified as a cell cycle regulatory element. By placing this region upstream of a reporter gene coding sequence a reporter cassette is potentially capable of signaling that the host cell population is about to undergo cell division in response to an external stimulus.

### Example 8; Hsp70 Reporter Gene Construct

The induction of heat shock genes (hsp) in response to stress serves to protect against the initial insult and produce a state of resistance to subsequent stress. This protective role is attributed to an active participation in the folding of proteins, maintenance of proteins in their native folding states and the repair or promotion of the degradation of mis-folded protein. Hsp70 is involved in cell apotosis induced by a variety of stress stimuli. It therefore potentially provides the basis for reporters capable of signaling a range of stress stimuli. It is induced by UV radiation, heat shock, heavy metals and pathological (infections, fever, inflammation, malignancy) or physiological (growth factors, hormonal stimulation tissue development) stimuli. A 5.0 Kb 5'-flanking region of mouse Hsp70 gene contains regulatory motifs shown to respond to acute stress by mediating increased transcription of the hsp70 gene. Reporters have therefore been engineered on this basis; the reporter coding sequence is placed under the control of a sequence starting ∼5kb upstream from the transcription start site of the hsp70 locus.

### Example 9; Hmox-1 Reporter Gene Construct

The HOZ reporter gene construct comprises regulatory sequences from the mouse haemoxygenase-1 (HO-1) gene operatively linked to LacZ, the coding sequence for β-galactosidase. HO-1 catalyses the initial reaction in the catabolism of haem to yield biliverdin and carbon monoxide and iron. Stimulation of HO-1 gene expression by most inducers is mediated at the level of transcription. Transcription is induced in response to oxidative stress, haem, heavy metals, heat shock and UV.

A number of previous studies have reported the derivation of clones that contained up to 14bk of upstream sequences from the mouse HO-1 gene (Alam, 1994; Alam et al., 1994; Alam et al., 1995; Zhang et al., 2001).

Several enhancer regions have been identified from these studies. The more distal of these, the AB1 region, is located 10kb upstream from the start site, indicating that at least 10kb of promoter sequence would be required for correct induction by various inducing agents. A further enhancer region, SX2, in located at -4kb, and several STAT binding sites at around -400 to -600bp. In addition, an enhancer element has been identified within the introns of the human gene, which regulates the haem and cadmium response (Hill-Kapturczak et al., 2003).

We have used RedET recombination cloning (Zhang et al., 2000) to produce a HO-1-LacZ reporter transgene. A LacZ-SV40polyA minigene was engineered to contain homologous regions to exons 1 and 2 of the HO-1 gene. This minigene cassette also contains a bacterial Amp gene, at the 3'-end, which was used as a selectable marker during cloning. The minigene was then introduced, by homologous recombination, into a BAC clone containing the mouse HO-1 locus (clone RPCI-23 290L07, HGMP Resource Centre) substituting for the endogenous HO-1 exon1 and intron1 sequences. Thus, the HO-1 promoter would express LacZ from the ATG start codon, in place of the HO-1 protein. The correct position of the LacZ-SV40polyA minigene was confirmed by sequencing both the 5'- and 3'-junction regions. The HO-1 reporter locus, containing most of the HO-1 gene together with 16.5kb of upstream promoter and 8kb of 3' sequence, was then recombined out from the BAC clone into the pACYC low copy number plasmid backbone.

Transient transfection of this construct into HEK293 cells, and induction with haemin resulted in an 11-fold increase in the LacZ labelling index over uninduced control cells.

Malstrom et al's HO-1 reporter uses 15kb of HO-1 sequence upstream of the transcription start site. The HOZ reporter gene of the present invention uses 16.5kb upstream and 8kb downstream of the transcriptional start site. The reporter of the present invention is therefore likely to contain regulatory DNA sequences absent from that used by Malstrom et al and its expression is therefore more likely to be regulated in a manner analogous of that of the endogenous HO-1 gene and therefore a better detector of cellular stress responses involving HO-1 gene activation.

### Example 10; PIG3 Reporter Gene Construct

p53 induced gene 3 or PIG3 was initially identified by serial analysis of gene expression (SAGE) as one of a panel of 14 transcripts whose expression was elevated more than 10 fold in p53 expressing colorectal cancer cells versus control cells (p53 null) (Polyak et al., 1997 Nature Genet. 30: 315-320, 2002). Reactive oxygen species (ROS) are potent inducers of apoptosis; PIG 3 encodes a quinone oxidoreductase homologue that may have a role in the metabolism of ROS.

Contente et al., (2002 Nature Genet. 30: 315-320) demonstrated that PIG3 transcription is upregulated in direct response to binding of p53 to a pentanucleotide repeat sequence with in the PIG 3 promoter (TGYCC)n where Y= C or T. This sequence is both necessary and sufficient for transcriptional activation of the PIG3 promoter. On this basis a construct comprising a reporter gene sequence placed downstream from the proximal promoter and the upstream sequences from the PIG3 gene, that contain the p53 recognition sequence repeats, potentially provides a reporter for p53 induction in the cells or tissue in which the reporter gene is present

### Example 11; SOD2 Reporter Gene Construct

Manganese superoxide dismutase (MnSOD, SOD2) is a member of a family of metalloenzymes that catalyze the dismutation of the superoxide anion to H2O2. The SOD2 gene encodes an intramitochondrial free radical scavenging enzyme that is the first line of defense against superoxide produced as a byproduct of oxidative phosphorylation. It is a tumor necrosis factor (TNF)-inducible gene product. It plays an important role in the generation of the intracellular signalling molecule H₂O₂.

The promoter region of the SOD2 gene contains a number of well characterized transcription factor recognition sequences including SP-1; AP-1; AP-2; NF-κB; STAT3; C/EBP; Egr-1; TNFRE. Transcription factors SP-1 and AP-2 seem to have opposite roles in the transcriptional activity of the basal promoter. Whereas SP-1 plays a positive role, which is absolutely essential for transcription from the human MnSOD promoter, AP-2 appears to play a negative role in this process. An enhancer element is found in the promoter region of the human MnSOD gene. Several important enhancer elements are located in the second intron. The NF-kappa B site in the second intron is essential but not sufficient for high-level induction of MnSOD by cytokines. Although mutations in the regulatory elements may be partially responsible for the lack of induction of MnSOD in some cell types, differences in the degree of induction exist that cannot be accounted for by the defect in the DNA sequence. It is highly likely that this difference is due to the presence or absence of coactivator or suppressor proteins in the cells and may have a physiological role in the defense against oxidative stress.

The human MnSOD promoter lacks both a TATA and a CAAT box but possesses several GC motifs. It has been shown that the proximal promoter region (basal promoter) contains multiple Sp1 and AP-2 binding sites and that Sp1 is essential for the constitutive expression of the MnSOD gene. An Egr-1 binding site has been identified in the basal promoter of MnSOD. The basal promoter is responsive to 12-O-tetradecanoylphorbol-13-acetate (TPA) in the human hepatocarcinoma cell line HepG2. The contributions of these binding sites and the roles of the transcription factors Egr-1, AP-2, and Sp1 in the activation of hMnSOD transcription by TPA have been investigated by site-directed mutation analysis, Western blotting, and overexpression of transcription factors. The results showed that Sp1 plays a positive role for both basal and TPA-activated hMnSOD transcription, whereas overexpression of Egr-1 has a negative role in the basal promoter activity without any effect on TPA-mediated activation of hMnSOD.

Reporter constructs containing reporter gene sequence cloned between -11.8kb of the 5'-promoter region and 18kb of gene plus the 3' region potentially provide a reporter for oxidative stress in the cells or tissue containing the reporter construct.

### Example 12; p21 - LacZ (WAZ44) reporter mice and effect of Etoposide

Injection of the pX3W construct into 431 fertilised mouse eggs as described resulted in a total of 96 offspring of which 12 were found to be transgenic by PCR analysis. Of these founder animals, six managed to successfully establish transgenic mouse lines. Of these, one line, WAZ44, was selected for detailed investigation based on the results of preliminary studies which indicated extremely low levels of basal transgene expression and transgene induction following toxic challenge.

Etoposide was selected as the compound for further evaluation with respect to LacZ induction in the WAZ44 mouse. Etoposide is a commonly used chemotherapeutic agent derived from epipodophyllotoxin whose mode of action as an anticancer agent involves inhibition of the enzyme topoisomerase II. Etoposide is a genotoxic carcinogen when administered chronically to mice. Its mode of action involves the induction of mitotic recombination leading to chromosomal rearrangements and loss of heterozygosity at key loci (Wijnhoven et al., 2003). Transgenic mice possessing an *E.coli* LacZ transgene which is only expressed after a DNA inversion involving the transgene have been used to demonstrate that etoposide causes significant induction of somatic intrachromosomal recombination events in vivo at doses between 0.05 and 50 mg/kg (Hooker et al., 2002, Sykes et al., 1999). Limited evidence does indicate that etoposide induces overt toxicity leading to chronic peritonitis and pleurisy in mice 3 - 4 weeks after *i.p.* administration of a toxic dose (Staehelin, 1976).

In experiments on WAZ44 mice, single doses of etoposide (of up to 40 mg/kg) were administered i.p. Animals were killed after 24 hours and tissues fixed and stained for LacZ expression. While little or no transgene expression was detected in control WAZ44 mice, animals that had received etoposide exhibited significant expression in the spleen, the thymus and in some regions of the brain.

### Example 13; p21 - LacZ (WAZ44) reporter mice and effect of Effects of Acrylamide

Acrylamide, at a total dose of 150 mg/kg, induces neuropathy manifesting as partial paralysis approximately 7 days after the start of treatment. The dose may be administered as a single injection or divided; however, the effect depends upon the total dose, and since a single dose of 150 mg/kg is likely to be acutely toxic, a five-day coursed of injections of 30 mg/kg is preferred. Acrylamide intoxication is associated with both central and peripheral effects; the central effects include behavioural changes (increased milk-licking) which occur within two days of dosing, preceding peripheral changes such as reduced hind-limb grip and reduced locomotor activity by up to three weeks (Teal and Evans, 1982). In experiments on WAZ44 mice, five doses of acrylamide (30 mg/kg) were administered i.p.

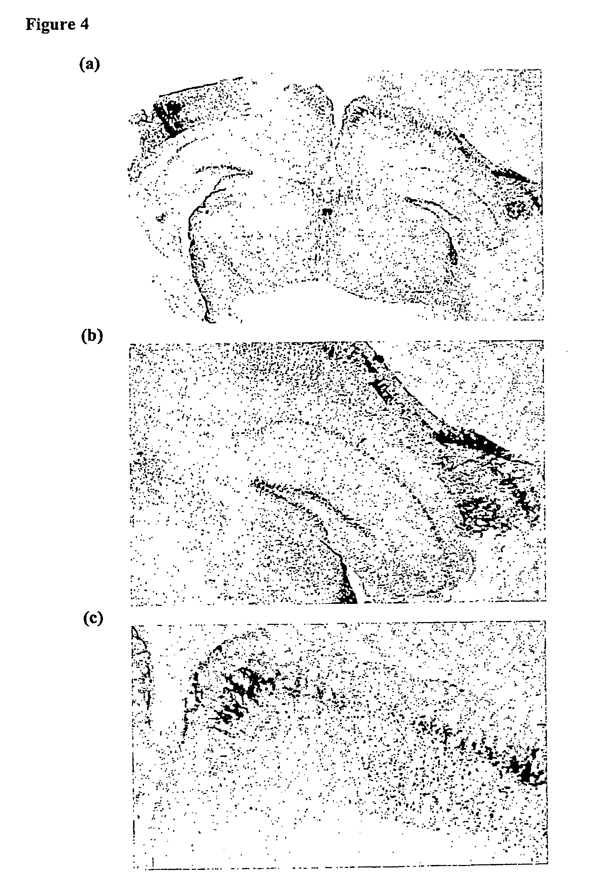

A frozen section from the brain of an acrylamide-treated WAZ44 mouse was prepared and stained using Frozen Method 1. A dissecting microscope was used to view the slides and generate photomicrographs (a) transverse section of whole brain, original magnification x10; (b) dentate gyrus and hippocampus, original magnification x 25 (c) cortex, original magnification x63. An example of results obtained using acrylamide is illustrated in Figure 4, which reveals a high level of LacZ expression in the dentate gyrus, hippocampus and cortex of acrylamide-treated WAZ44 mouse brain.

### Example 14; WAZ Transgene Expression in Brain.

To further investigate WAZ gene induction in the brain, we compared the effects of etoposide with those of mercuric chloride (HgCl₂) which has been shown to induce p21 (Bartosiewicz et al,. 2001), lipopolysaccharide (LPS) which has been shown to induce p21 in the hippocampus (Ring et al,. 2002), and paracetamol which is known to cross the blood-brain barrier.

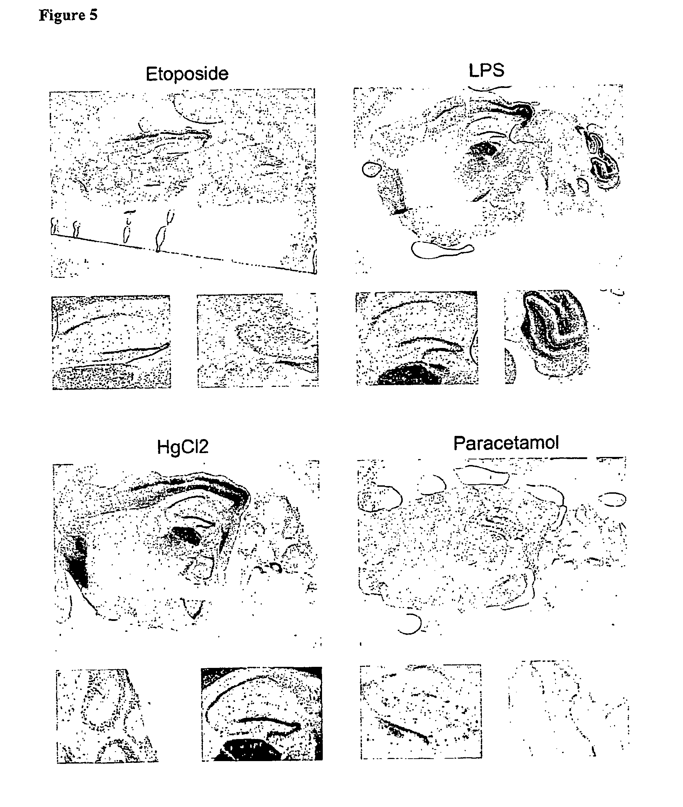

WAZ mice received single i.p. injections of etoposide (40mg/kg); mercuric chloride (HgCl₂, 8.5mg/kg); lipopolysaccharide (LPS, 1.5mg/kg) or paracetamol (300mg/kg). WAZ mice received a single i.p. injection of each compound at a single dose level (see Figure 5). Twenty-four hours later, the mice were killed and the brains sectioned and stained for LacZ expression. Figure X. shows the LacZ staining resulting from administration of each compound. All compounds induced high levels of expression of the WAZ transgene in the brain. Etoposide, mercuric chloride and LPS all induced expression in the same cells within the various regions in the brain. However, paracetamol produced a distinct pattern of induction within the hippocampus, cerebellum and cortex.

### Example 15; HO-1-LacZ (HOZ) reporter mice and Effects of cadmium chloride

The HOZ mouse contains a LacZ reporter gene driven by the HO-1 promoter described under Example 9. The HO-1-LacZ transgene was inserted into the murine Hprt gene (X chromosome), by homologous recombination, using the BPES cell line as described under 'Targeted Transgenesis'.

Two ES cell clones were selected for injection into blastocysts and 4 male chimaeric mice were subsequently obtained from one of the clones. Three of the chimaeras were 100% ES derived and these male animals were bred to B6 females to produce 24 offspring. These animals were then used to examine the inducibility of the HOZ transgene reporter.

Preliminary characterisation of the HOZ transgene response to toxic insult was performed with cadmium chloride. Following oral administration of cadmium chloride, cadmium is absorbed via the proximal duodenum and transported to the liver, where it is deposited before being redistributed to the kidneys (Sorensen et al., 1993). Accordingly, cadmium causes acute hepatotoxicity followed by chronic nephrotoxicity. The nephrotoxic effect of cadmium is thought to be due to renal uptake of metallothionein-Cd which is synthesised in the liver following acute exposure and released into the circulation (Sendelbach and Klaassen, 1988).

The biochemical toxicity of cadmium involves a number of changes related to oxidative stress, including enhanced hepatic lipid peroxidation, glutathione depletion, upregulation of γ-glutamyl transpeptidase, and down-regulation of GSTs and CYPs (Andersen and Andersen, 1988, Dalvi and Robbins, 1978, Karmakar et al., 1999).

Initially, one HOZ mouse was given 10 mg/kg body weight cadmium chloride i.p. One control animal received the vehicle solution, isotonic saline, i.p. and an untreated animal was used as a second control. The animals were killed 24 hours after dosing and liver, kidney and brain were collected for H&E staining and *lacZ* histology.

Subsequently, a further experiment was carried out to extend these findings using a larger number of HOZ mice. For this experiment, both male and female HOZ mice were used and the effects of cadmium chloride in the dose range 2-8 mg/kg at 8 and 24 hours after administration were examined. The doses selected were based on previous experience and on literature reports that these doses cause HO-1 induction in mouse liver without inducing unacceptable toxicity (Aleksunes et al., 2005, Kenyon et al., 2005, Malstrom et al., 2004)

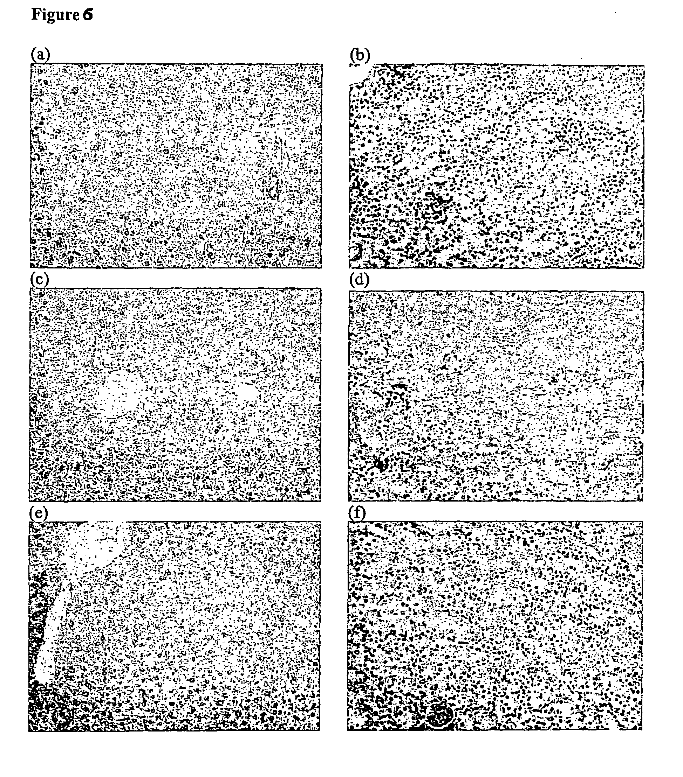

Sections of HOZ mouse liver and kidney were prepared and stained according to standard procedures (H&E). (a, b) Naive control; (c, d) Saline treated; (e, f) Cadmium chloride treated (10 mg/kg i.p. in saline). All images in Figure 5 are of H&E stained sections: (a, c and e) are of liver; b, d and f are of kidney (original magnification x 100). Evaluation of H&E stained sections indicated no marked hepatic abnormalities in naive and vehicle-treated control animals, whereas the appearance of liver from the cadmium chloride treated animal indicated acute toxicity manifesting as hepatocyte vacuolation and areas of necrosis associated with deposits of erythrocytes (Figure 6, a, c and e). Histological evaluation of the kidneys revealed the presence of occasional necrotic proximal convoluted tubules (PCTs) in the cadmium chloride-treated mouse kidney whereas no marked abnormalities were observed in naive or saline-treated control kidneys (Figure 6, b, d and f).

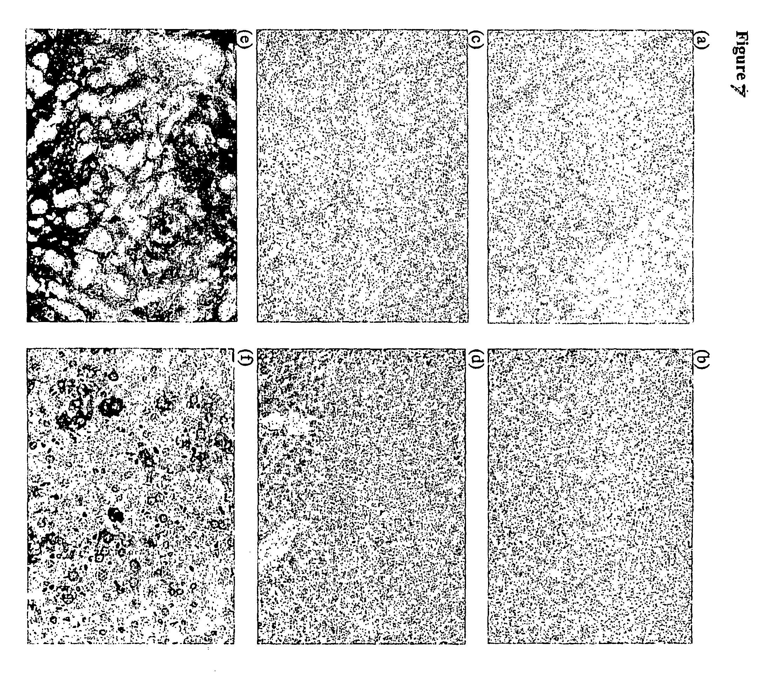

Sections of HOZ mouse liver were prepared and stained as described. (a, b) Naive control; (c, d) Saline treated; (e, f) Cadmium chloride treated (10 mg/kg i.p. in saline). The sections shown in images a, c and e were frozen according the Frozen Method 1 and stained according to Frozen Method 2 (original magnification x 200) and those shown in b, d and f were prepared and stained by Whole Mount Method (original magnification x200). The livers of the naive and vehicle-treated control animals were devoid of LacZ staining (Figure 7, a, b, c and d). In contrast, intense blue staining indicating expression of LacZ was observed in the liver of the cadmium chloride-treated mouse 24 hours after dosing (Figure 7, e and f). The staining appeared to be most intense around the central veins of the liver lobules.

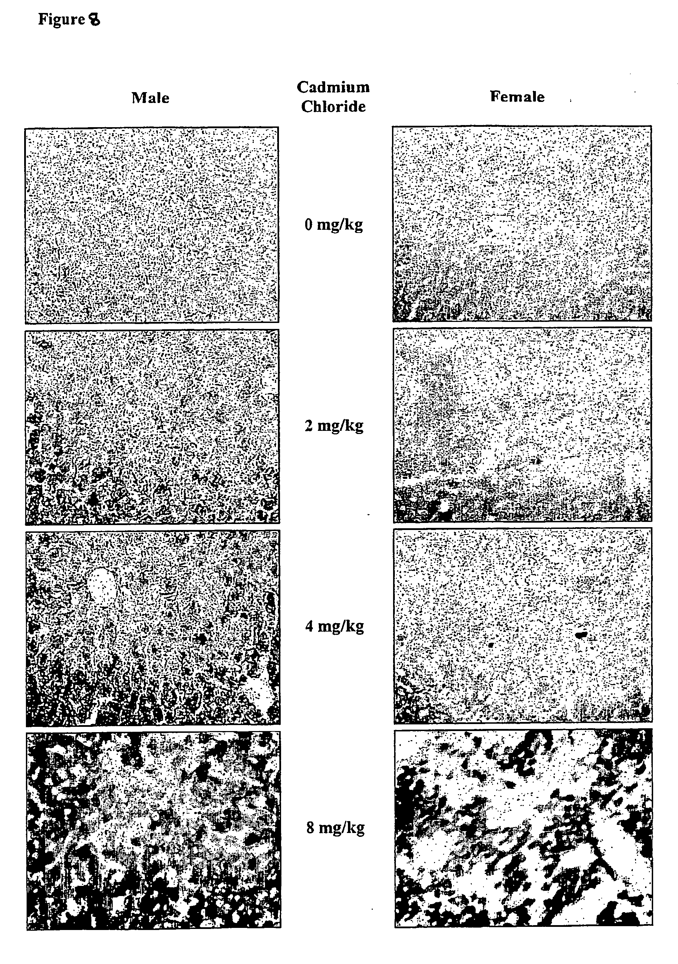

With reference to Figure 8, the response is shown of HOZ transgene expression in HOZ mouse liver 24 hours after administration of various does of cadmium chloride. HOZ mice of each sex were given intra-peritoneal injections (10ml/kg body weight) of isotonic saline containing cadmium chloride at concentrations calculated to yield a total dose per animal of 0, 2, 4 or 8 mg/kg body weight as indicated. After 24 hours, animals were killed and sections of liver tissue stained for LacZ. When the effects of lower cadmium chloride doses were examined, HOZ gene expression was clearly apparent in livers from animals of either sex 24 hours after 8mg/kg cadmium chloride. In male animals, some HOZ expression was also apparent following lower doses of cadmium chloride, but none was evident in female animals (Figure 8).

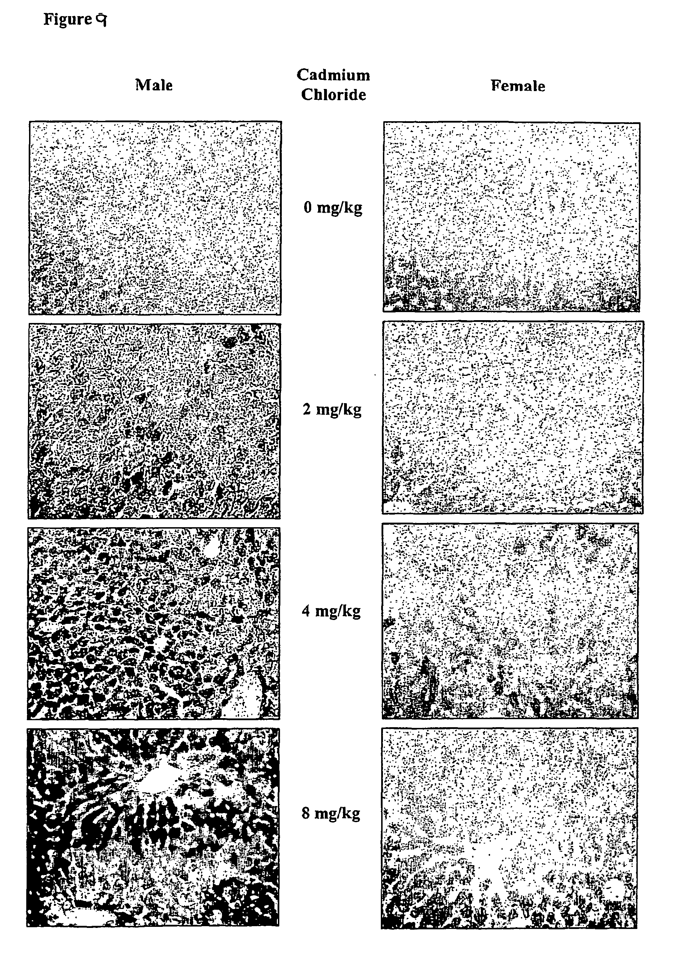

With reference to Figure 9, there is shown the response of HOZ transgene expression in HOZ mouse liver 8 hours after administration of various does of cadmium chloride. HOZ mice of each sex were given intra-peritoneal injections (10ml/kg body weight) of isotonic saline containing cadmium chloride at concentrations calculated to yield a total dose per animal of 0, 2, 4 or 8 mg/kg body weight as indicated. After 8 hours, animals were killed and sections of liver tissue stained for LacZ. High levels of HOZ gene expression were also apparent in liver from male mice only 8 hours following 8mg/kg cadmium chloride with lower levels of expression in animals that had received lower doses. In livers from female animals, there was no consistent evidence of HOZ expression at 8 hours, although weak LacZ staining was observed in the animal that had received 4 mg/kg cadmium chloride (Figure 9).

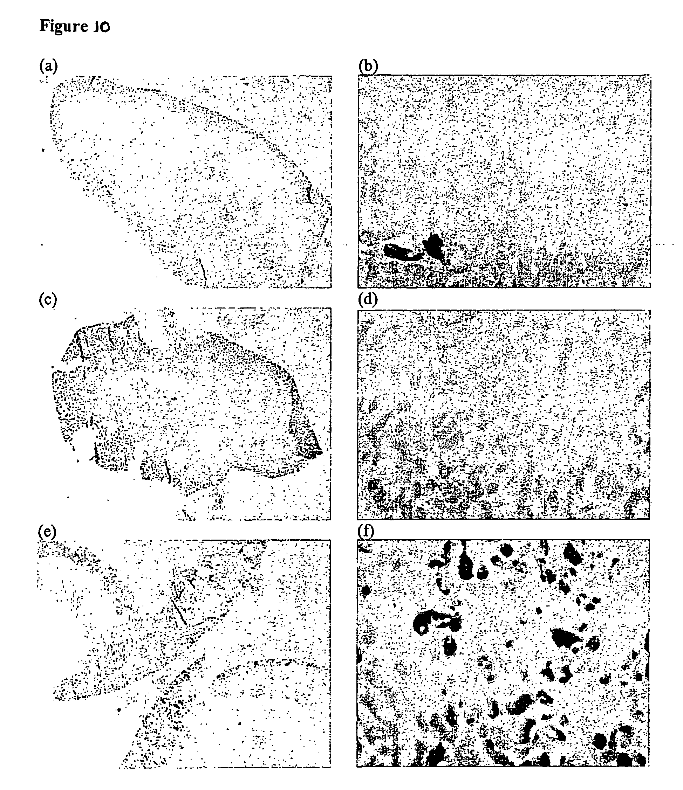

When LacZ staining was evaluated in the kidney, punctate blue staining could be seen (Figure 10). Sections of HOZ mouse kidney were prepared and stained as described. (a, b) Naive control; (c, d) Saline treated; (e, f) Cadmium chloride treated (10 mg/kg i.p. in saline). The sections were frozen according the Frozen Method 1 and stained according to Frozen Method 2 (b, e and h: original magnification x10; c, f and i: original magnification x 200)In naive and saline treated control animals, only a few proximal convoluted tubules (PCTs) were stained (Figure 10, a, b, c and d) whereas in the cadmium chloride-treated mouse the frequency of stained PCTs was much higher (Figure 10, e and f). This pattern was confirmed in a subsequent experiment in which 8 mg/kg cadmium chloride produced a detectable expression of the HOZ gene in kidney 8 hours after dosing.

These results show induction of the HOZ transgene in kidney in response to cadmium chloride.

There was no evidence of LacZ expression in the brains of HOZ mice, with or without cadmium chloride treatment.

The data from Malstrom et al Ho1-luc transgenic mouse studies report that their luciferase signal in response to cadmium chloride was only "moderately" correlated with alanine aminotransferase (ALT) levels. In contrast, our results show HOZ transgene expression at lower doses of cadmium chloride before ALT elevation is evident. HOZ transgene expression is therefore a predictor of toxicity rather than merely being correlated with it. This evidence suggests that the system of the present invention is not only more accurate but is more sensitive than the prior art methods.

### Example 16; Expression of Endogenous HO-1

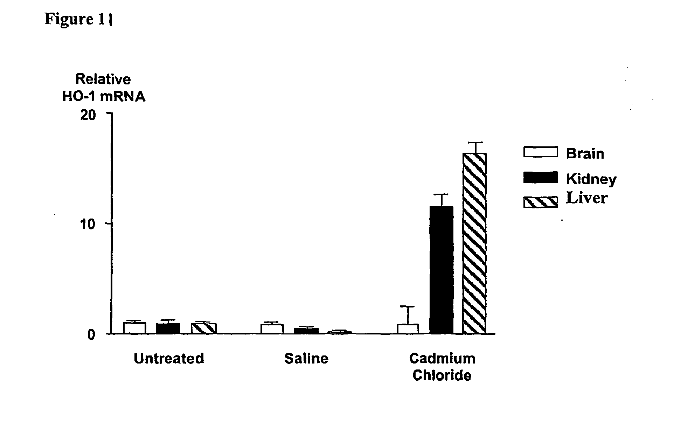

The effects of cadmium chloride on endogenous HO-1 expression were determined by RT-PCR assay of HO-1 mRNA levels in liver and kidney tissue from HOZ mice. Figure 11 shows HO-1 mRNA levels in HOZ mouse liver 24 hours after administration of 10 mg/kg cadmium chloride. The bars show relative concentrations of HO-1 mRNA assayed by RT-PCR in brain, kidney and liver tissue of mice that had received 10 mg/kg cadmium chloride 24 hours earlier compared with mice that had received saline and untreated mice.

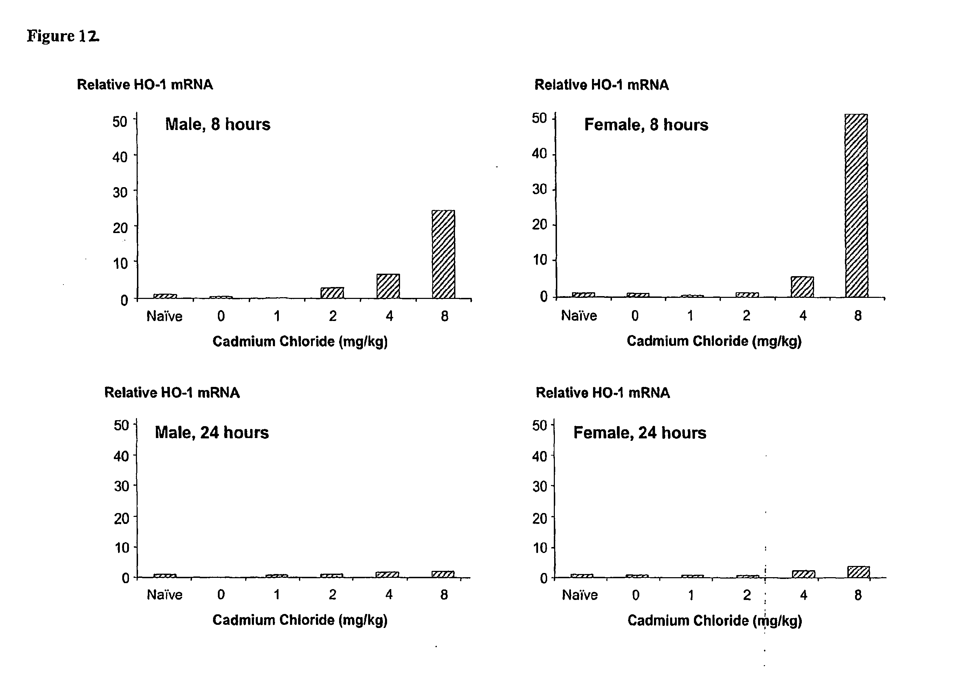

In animals that had received 10mg/kg cadmium chloride, HO-1 mRNA-levels in the kidney and liver after 24 hours were very substantially elevated compared to controls (Figure 11). In liver, a clear dose-response relationship between increasing dose of cadmium chloride and increasing HO-1 mRNA was evident in both sexes at 8 hours. This had all but disappeared by 24 hours although HO-1 mRNA levels were still slightly elevated in the livers of animals that had received the highest doses of cadmium chloride (Figure 12). With reference to Figure 12, HO-1 mRNA levels in HOZ mouse liver 8 hours and 24 hours after administration of various does of cadmium chloride are shown. The bars indicate relative concentrations of HO-1 mRNA assayed by RT-PCR in HOZ mouse liver following doses of cadmium chloride ranging from 0 - 8 mg/kg body weight. The panels compare the effects seen in male and female mice at 8 or 24 hours after cadmium chloride injection.

### Example 17; Effects of Sodium Arsenite

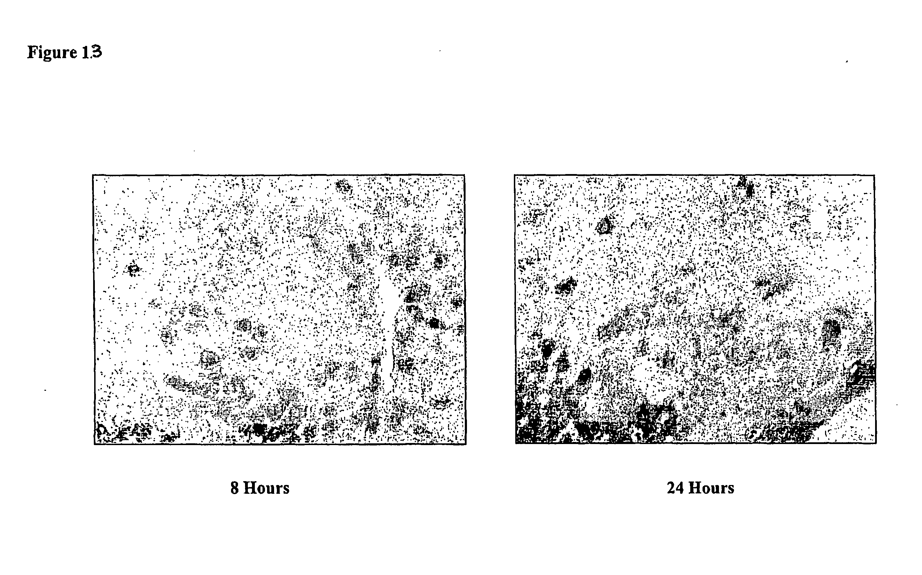

Previous reports on the induction of HO-1 by sodium (meta)arsenite indicated that HO-1 activity peaked at 8 hours following i.p. adminstration. We therefore examined the effects of sodium arsenite administration on HOZ transgene expression at 8 and 24 hours after dosing. Female HOZ mice received sodium arsenite at 13 mg/kg body weight in isotonic saline, i.p. and after either 8 hours or 24 hours, liver tissue was collected and stained for LacZ expression. These results indicated expression of the HOZ transgene at both time points (Figure 13).

### Example 18; Markers of Hepatotoxicity and Nephrotoxicity

Two plasma enzyme markers, ALT and AST, were used as markers of hepatotoxicity in HOZ mice treated with cadmium chloride or sodium arsenite. The results of this analysis for HOZ mice treated with 10 mg/kg cadmium chloride are shown in Table 1 and for HOZ mice treated with 2-8 mg/kg cadmium chloride or 13 mg/kg sodium arsenite in Tables 2 and 3.

**Table 1: Plasma enzyme markers in naive, vehicle control and cadmium chloride-treated HOZ mice**

| | | **U/L** | |
|---|---|---|---|
| **Mouse ID** | **Treatment** | **ALT** | **AST** |
| 1 | Untreated | 45 | 119.1 |
| 2 | Saline | 33 | 104.2 |
| 3 | 10mg/kg Cadmium Chloride | 1834.8 | 4643.1 |

**Table 2: Plasma ALT (u/L) as a marker of hepatotoxicity in HOZ mice treated**

**with HO-1 inducers**

| **Sex** | **Treatment** | **8 hours** | **24 hours** |
|---|---|---|---|
| Male | None | 87.8 | 39.0 |
| | Saline | 30.5 | 36.9 |
| | 1mg/kg Cadmium Chloride | 70.3 | 33.1 |
| | 2mg/kg Cadmium Chloride | 21.7 | 19.4 |
| | 4mg/kg Cadmium Chloride | 50.4 | 74.7 |
| | 8mg/kg Cadmium Chloride | 50.1 | 306.3 |
| Female | None | 23.1 | 25.5 |
| | Saline | 39.0 | 35.7 |
| | 1mg/kg Cadmium Chloride | 33.3 | 28.2 |
| | 2mg/kg Cadmium Chloride | 22.8 | 26.1 |
| | 4mg/kg Cadmium Chloride | 55.6 | 23.7 |
| | 8mg/kg Cadmium Chloride | 3930.6 | 7010.4 |
| | 13mg/kg Sodium Arsenite | 43.0 | 26.7 |

**Table 3: Plasma AST (u/L) as a marker of hepatotoxicity in HOZ mice treated with HO-1 inducers**

| **Sex** | **Treatment** | **8 hours** | **24 hours** |
|---|---|---|---|
| Male | None | 142.0 | 92.1 |
| | Saline | 65.0 | 84.0 |
| | 1mg/kg Cadmium Chloride | 100.2 | 63.1 |
| | 2mg/kg Cadmium Chloride | 66.4 | 56.4 |
| | 4mg/kg Cadmium Chloride | 132.3 | 123.0 |
| | 8mg/kg Cadmium Chlorine | 92.4 | 353.8 |
| Female | None | 56.7 | 83.4 |
| | Saline | 73.8 | 89.4 |
| | 1mg/kg Cadmium Chloride | 87.6 | 87.5 |
| | 2mg/kg Cadmium Chloride | 80.4 | 104.7 |
| | 4mg/kg Cadmium Chloride | 108.5 | 89.1 |
| | 8mg/kg Cadmium Chloride | 13425.6 | 8097.6 |
| | 13mg/kg Sodium Arsenite | 107.3 | 67.8 |

Female HOZ mice exhibited a marked and rapid response to cadmium chloride at 8 mg/kg. The increase in plasma ALT was 170-fold after 8 hours and 275-fold after 24 hours, while the corresponding increases for AST were 237-fold after 8 hours and 97-fold after 24 hours. The dose response was steep: the increase in plasma ALT in response to a 4 mg/kg dose of cadmium chloride was only 2.4-fold after 8 hours and there was no increase above 2-fold in ALT after 8 hours, while no increase in plasma AST was observed at either time point. The response of male HOZ mice to cadmium chloride was slower and more muted, with a 7.9-fold increase in ALT and a 3.8-fold increase in AST 24 hours after treatment with 8 mg/kg but otherwise no increase above 2-fold. HOZ mice did not exhibit a hepatotoxic response to sodium arsenite.

Nephrotoxicity was estimated by measuring the blood urea nitrogen (BUN) levels as shown in Table 4. Female mice exhibited mild nephrotoxicity, as measured by BUN, in response to cadmium chloride at 8 mg/kg. The increase in BUN was 6-fold at 8 hours and 44-fold after 24 hours. No nephrotoxicity, as measured using this marker, was observed in male mice.

**Table 4: BUN as a marker of nephrotoxicity in HOZ mice treated with HO-1 inducers**

| **Sex** | **Treatment** | **8 hours** | **24 hours** |
|---|---|---|---|
| Male | None | 4.26 | 4.77 |
| | Saline | 6.61 | 6.09 |
| | 1mg/kg Cadmium Chloride | 4.54 | 5.27 |
| | 2mg/kg Cadmium Chloride | 4.24 | 5.10 |
| | 4mg/kg Cadmium Chloride | 3.87 | 4.11 |
| | 8mg/kg Cadmium Chloride | 3.84 | 4.85 |
| Female | None | 3.81 | 2.22 |
| | Saline | 2.82 | 3.82 |
| | 1mg/kg Cadmium Chloride | 5.10 | 4.36 |
| | 2mg/kg Cadmium Chloride | 3.33 | 2.85 |
| | 4mg/kg Cadmium Chloride | 4.77 | 3.66 |
| | 8mg/kg Cadmium Chloride | 22.86 | 98.16 |
| | 13mg/kg Sodium Arsenite | 5.62 | 3.50 |

### References

Alam, J. (1994). Multiple elements within the 5' distal enhancer of the mouse heme oxygenase-1 gene mediate induction by heavy metals. J Biol Chem 269, 25049-25056.
Alam, J., Cai, J., and Smith, A. (1994). Isolation and characterization of the mouse heme oxygenase-1 gene. Distal 5' sequences are required for induction by heme or heavy metals. J Biol Chem 269, 1001-1009.
Alam, J., Camhi, S., and Choi, A. M. (1995). Identification of a second region upstream of the mouse heme oxygenase-1 gene that functions as a basal level and Inducer-dependent transcription enhancer. J Biol Chem 270, 11977-11984.
Aleksunes, L. M., Sliit, A. M., Cherrington, N. J., Thibodeau, M. S., Klaassen, C. D. and Manautou, J. E. (2005) Differential expression of mouse hepatic transporter genes in response to acetaminophen and carbon tetrachloride Toxicological Sciences, 83, 44-52.
Andersen, H. R. and Andersen, O. (1988) Effect of cadmium chloride on hepatic lipid peroxidation in mice Pharmacology and Toxicology, 63, 173-177.
Bartosiewicz, M. Jenkins, D. Penn, S. Emery, J. Buckpitt, A. (2001). Unique gene expression patterns in Liver and Kidney associated with exposure to chemical toxicants. The Journal of Pharmacology and Experimental Therapeutics, 293, 895-905.
Campbell, S. J., Carlotti, F., Hall, P. A., Clark, A. J., and Wolf, C. R. (1996). Regulation of the CYP1A1 promoter in transgenic mice: an exquisitely sensitive on-off system for cell specific gene regulation. J Cell Sci 109 ( Pt 11), 2619-2625.
Campbell, S. J., Henderson, C. J., Anthony, D. C., Davidson, D., Clark, A. J. and Wolf, C. R. (2005) The murine cyp1a1 gene is expressed in a restricted spatial and temporal pattern during embryonic development Journal of Biological Chemistry, 280, 5828-5835.
Dalvi, R. R. and Robbins, T. J. (1978) Comparative studies on the effect of cadmium, cobalt, lead, and selenium on hepatic microsomal monooxygenase enzymes and glutathione levels in mice Journal of Environmental Pathology and Toxicology, 1, 601-607.
Farhadi, H. F., Lepage, P., Forghani, R., Friedman, H. C., Orfali, W., Jasmin, L., Miller, W., Hudson, T. J., and Peterson, A. C. (2003). A combinatorial network of evolutionarily conserved myelin basic protein regulatory sequences confers distinct glial-specific phenotypes. J Neurosci 23, 10214-10223.
Heaney, J. D., Rettew, A. N., and Bronson, S. K. (2004). Tissue-specific expression of a BAC transgene targeted to the Hprt locus in mouse embryonic stem cells. Genomics 83, 1072-1082.
Hill-Kapturczak, N., Sikorski, E., Voakes, C., Garcia, J., Nick, H. S., and Agarwal, A. (2003). An internal enhancer regulates heme- and cadmium-mediated induction of human heme oxygenase-1. Am J Physiol Renal Physiol 285, F515-523.
Hooker, A. M., Home, R., Morley, A. A. and Sykes, P. J. (2002) Dose-dependent increase or decrease of somatic intrachromosomal recombination produced by etoposide Mutation Research - Fundamental and Molecular Mechanisms of Mutagenesis, 500, 117-124.
Karmakar, R., Roy, S. and Chatterjee, M. (1999) The effects of cadmium on the hepatic and renal levels of reduced glutathione, the activity of glutathione s-transferase and gamma glutamyl transpeptidase Journal of Environmental Pathology, Toxicology and Oncology, 18, 29-35.
Kenyon, E. M., Del Razo, L. M., Hughes, M. F. and Kitchin, K. T. (2005) An integrated pharmacokinetic and pharmacodynamic study of arsenite action: 2. Heme oxygenase induction in mice Toxicology, 206, 389-401.
Malstrom, S. E., Jekic-Mcmullen, D., Sambucetti, L., Ang, A., Reeves, R., Purchio, A. F., Contag, P. R. and West, D. B. (2004) In vivo bioluminescent monitoring of chemical toxicity using heme oxygenase-luciferase transgenic mice Toxicology and Applied Pharmacology, 200, 219-228.
Ring, R. Valo, Z. Gao, C. Barish, M. Singer-Sam, J. (2003). The cdkn1a gene (p21WAF1/CIP1) is an inflammatory response gene in the mouse central nervous system. Neuroscience Letters 350, 73-76.
Sendelbach, L. E. and Klaassen, C. D. (1988) Kidney synthesizes less metallothionein than liver in response to cadmium chloride and cadmium-metallothionein Toxicology and Applied Pharmacology, 92, 95-102.
Sorensen, J. A., Nielsen, J. B. and Andersen, O. (1993) Identification of the gastrointestinal absorption site for cadmium chloride in vivo Pharmacology and Toxicology, 73, 169-173.
Staehelin, H. (1976) Delayed toxicity of epipodophyllotoxin derivatives (vm 26 and vp 16-213), due to a local effect European Journal of Cancer and Clinical Oncology, 12, 925-931.
Sun, X, Zhou, Z. and Kang, Y.J. (2001) Attenuation of Doxorubicin Chronic Toxicity in Metallothionein-overexpressing Transgenic Mouse Heart, Cancer Research 61, 3382-3387.
Sykes, P. J., Hooker, A. M. and Morley, A. A. (1999) Inversion due to intrachromosomal recombination produced by carcinogens in a transgenic mouse model Mutation Research - Fundamental and Molecular Mechanisms of Mutagenesis, 427, 1-9.
Teal, J. J. and Evans, H. L. (1982) Behavioral effects of acrylamide in the mouse Toxicology and Applied Pharmacology, 63,470-480.
Wijnhoven, S. W. P., Sonneveld, E., Kool, H. J. M., van Teijlingen, C. M. M. and Vrieling, H. (2003) Chemical carcinogens induce varying patterns of loh in mouse t-lymphocytes Carcinogenesis, 24, 139-144.
Zhang, W., Feng, J. Q., Harris, S. E., Contag, P. R., Stevenson, D. K., and Contag, C. H. (2001). Rapid in vivo functional analysis of transgenes in mice using whole body imaging of luciferase expression. Transgenic Res 10, 423-434.
Zhang, Y., Muyrers, J. P., Testa, G., and Stewart, A. F. (2000). DNA cloning by homologous recombination in Escherichia coli. Nat Biotechnol 18, 1314-1317.

## Claims

1. A nucleic acid construct comprising (*i*) a nucleic acid sequence of a promoter region of Hmox-1 comprising 16.5kb upstream and 8kb downstream from the transcription start site of a mammalian Hmox-1 gene and (*ii*) a nucleic acid sequence comprising a transcriptional start site and (*iii*) at least one or more nucleic acid sequence capable of acting as a template for a defined RNA transcript and providing a read-out in the form of an excretable protein and/or an agent capable of being detected histologically.

2. A nucleic acid sequence according claim 1 wherein the nucleic acid sequence capable of acting as a template for a defined RNA transcript or reporter sequence is selected to provide a convenient read out of gene expression through assay of either the transcript or the encoded translation product polypeptide.

3. A nucleic acid construct according to claim 2 wherein transcription of the reporter sequence is detected by any one or more of the following:
(i) assay of the RNA transcript of the reporter sequence or;
(ii) assay of a polypeptide translation product of the reporter sequence.

4. A nucleic acid construct according to claim 1 wherein the nucleic acid sequence of a promoter region of Hmox-1 is operatively isolated from a transcriptional start site either by (i) a nucleotide isolator sequence that is flanked on either side by nucleic acid sequences recognised by a site specific recombinase, or (ii) by insertion of the promoter such that it is inverted with respect to the nucleic acid sequence comprising a transcriptional start site and the nucleic acid sequence capable of acting as a template for a defined RNA transcript and wherein the inverted promoter is positioned between nucleic acid sequences recognised by a site specific recombinase.

5. A nucleic acid construct according to claim 4 wherein the recombinase recognition sites are arranged in such a way that the nucleotide isolator sequence is deleted or the inverted promoter sequence's orientation is reversed in the presence of the recombinase.

6. A nucleic acid construct according to either claim 4 or 5 further comprising a nucleic acid sequence comprising a tissue-specific promoter operatively linked to a gene encoding a coding sequence for the site-specific recombinase.

7. A nucleic acid construct according to any of claims 4 to 6 wherein the site-specific recombinase system is the bacteriophage P1 cre-lox system using two *lox*P - sites or a bacterial FLIP system.

8. A host cell transfected with a nucleic acid construct according to any preceding claim which expresses a protein encoded by the nucleic acid construct.

9. A host cell according to claim 8 that is subjected to further transgenesis, in which the transgenesis is the introduction of an additional gene or genes or protein-encoding nucleic acid sequence or sequences.

10. A transgenic non-human animal including a nucleic acid construct according to any of claims 1 to 7 in which cells of the non-human animal express a protein encoded by the nucleic acid construct.

11. A transgenic non-human animal according to claim 10 that is subjected to further transgenesis, in which the transgenesis is the introduction of an additional gene or genes or protein-encoding nucleic acid sequence or sequences.

12. Use of a nucleic acid construct according to any of claims 1 to 7 for the detection of a gene activation event resulting from a toxic insult in a hepatic or renal cell *in vitro* and optionally wherein the gene activation event is signalled by expression of a reporter sequence whose translation product is identified by means of an epitope tag peptide sequence.

13. Use according to claim 12 wherein the nucleic acid sequence capable of acting as a template for a defined reporter sequence or RNA transcription and/or translation products are heterologous to the cell in which the reporter sequence is expressed.,

14. A method of screening for, characterising or monitoring cellular stress responses involving Hmox-1 gene activation in a hepatic or renal cell or cell line according to either of claims 8 or 9 or in the liver or kidney of a non human transgenic animal according to either of claims 10 or 11 comprising exposing the cell, cell line or transgenic non human animal to toxicologically induced stress and monitoring reporter or read-out product(s).

15. A host cell according to either of claims 8 or 9 further transfected with one or more additional nucleic acid constructs comprising:
(a) a promoter region of a gene or set of genes selected from the group consisting of a p21, metallothionein 1A, PUMA, Gclc, Cox2, Ki67, Stk6, Hsp, PIG3 and SOD2 gene and whose expression is modified in response to an adverse extracellular or intracellular change in the cellular environment selected from the group consisting of
(i) disturbances in the homeostatic state of DNA in the cell;
(ii) changes in response to oxidative stress of a cell;
(iii) changes that cause hepatotoxic stress;
(iv) stimuli that trigger cellular apoptosis;
(v) administration of chemicals, therapeutics or other xenobiotic agents and;
(vi) disease states whether natural, induced or modelled; and
(b) a nucleic acid sequence comprising a transcriptional start site; and
(c) at least one or more nucleic acid sequence capable of acting as a template for a defined RNA transcript and providing a read-out in the form of an excretable protein and/or an agent capable of being detected histologically.

16. A host cell according to claim 15 wherein the promoter region comprises any one or more of the following promoter sequences:
(i) up to 5 kb from a region immediately 5' to the transcription start site of a mammalian p21 gene;
(ii) up to 26 kb of an upstream segment of a mammalian metallothionein 1A gene;
(iii) up to 16 kb of an upstream gene sequence and a 5kb segment of downstream sequence of a mammalian PUMA gene;
(iv) up to 16kb of an upstream gene sequence of a mammalian Gclc gene;
(v) up to 3.5kb sequence upstream from the transcriptional start site and a further 3kb segment from the coding sequences of a mammalian cox 2 gene;
(vi) up to 30kb of an upstream gene sequence of the transcriptional start of a mammalian Ki67 gene;
(vii) an E4TF1 motif (-87 to -78) and an Sp-1 site (-129 to -121) and optionally a tandem repressor element CDE/CHR(-53 to -49/-39 to -35) of a mammalian Stk6 gene;
(viii) up to 5kb of 5' flanking region of a mammalian Hsp70 gene;
(ix) p53 recognition sequence repeats of a mammalian PIG3 gene; and
(x) up to 11.8kb of the 5' promoter region and 18 kb of a mammalian SOD2 gene and the 3'region of a mammalian SOD2 gene.

17. A transgenic non-human animal according to either of claims 10 or 11 further including one or more nucleic acid constructs comprising:
(a) a promoter region of a gene or set of genes selected from the group consisting of a p21, metallothionein 1A, PUMA, Gclc, Cox2, Ki67, Stk6, Hsp, PIG3 and SOD2 gene and whose expression is modified in response to an adverse extracellular or intracellular change in the cellular environment selected from the group consisting of
(i) disturbances in the homeostatic state of DNA in the cell;
(ii) changes in response to oxidative stress of a cell;
(iii) changes that cause hepatotoxic stress;
(iv) stimuli that trigger cellular apoptosis;
(v) administration of chemicals, therapeutics or other xenobiotic agents and;
(vi) disease states whether natural, induced or modelled; and
(b) a nucleic acid sequence comprising a transcriptional start site; and
(c) at least one or more nucleic acid sequence capable of acting as a template for a defined RNA transcript and providing a read-out in the form of an excretable protein and/or an agent capable of being detected histologically.

18. A transgenic non-human animal according to claim 17 wherein the promoter region comprises any one or more of the following promoter sequences:
(i) up to 5 kb from a region immediately 5' to the transcription start site of a mammalian p21 gene;
(ii) up to 26 kb of an upstream segment of a mammalian metallothioneien 1A gene;
(iii) up to 16 kb of an upstream gene sequence and a 5kb segment of downstream sequence of a mammalian PUMA gene;
(iv) up to 16kb of an upstream gene sequence of a mammalian Gclc gene;
(v) up to 3.5kb sequence upstream from the transcriptional start site and a further 3kb segment from the coding sequences of a mammalian cox 2 gene;
(vi) up to 30kb of an upstream gene sequence of the transcriptional start of a mammalian Ki67 gene;
(vii) an E4TF1 motif (-87 to -78) and an Sp-1 site (-129 to -121) and optionally a tandem repressor element CDE/CHR(-53 to -49/-39 to -35) of a mammalian Stk6 gene;
(viii) up to 5kb of 5' flanking region of a mammalian Hsp70 gene;
(ix) p53 recognition sequence repeats of a mammalian PIG3 gene; and
(x) up to 11.8kb of the 5' promoter region and 18 kb of a mammalian SOD2 gene and the 3'region of a mammalian SOD2 gene.

19. A method according to claim 14 wherein cells or cell lines or non-human transgenic animals are transfected with or carry a Hmox-1 nucleic acid construct and at least one further different nucleic acid construct comprising:
(a) a promoter region of a gene or set of genes selected from the group consisting of a p21, metallothionein 1A, PUMA, Gclc, Cox2, Ki67, Stk6, Hsp, PIG3 and SOD2 gene and whose expression is modified in response to an adverse extracellular or intracellular change in the cellular environment selected from the group consisting of
(i) disturbances in the homeostatic state of DNA in the cell;
(ii) changes in response to oxidative stress of a cell;
(iii) changes that cause hepatotoxic stress;
(iv) stimuli that trigger cellular apoptosis;
(v) administration of chemicals, therapeutics or other xenobiotic agents and;
(vi) disease states whether natural, induced or modelled; and
(b) a nucleic acid sequence comprising a transcriptional start site; and
(c) at least one or more nucleic acid sequence capable of acting as a template for a defined RNA transcript and providing a read-out in the form of an excretable protein and/or an agent capable of being detected histologically.
so that a variety of different reporter or read-out product(s) can be monitored simultaneously.

20. A method according to claim 19 wherein the promoter region comprises any one or more of the following promoter sequences:
(i) up to 5 kb from a region immediately 5' to the transcription start site of a mammalian p21 gene;
(ii) up to 26 kb of an upstream segment of a mammalian metallothioneien 1A gene;
(iii) up to 16 kb of an upstream gene sequence and a 5kb segment of downstream sequence of a mammalian PUMA gene;
(iv) up to 16kb of an upstream gene sequence of a mammalian Gclc gene;
(v) up to 3.5kb sequence upstream from the transcriptional start site and a further 3kb segment from the coding sequences of a mammalian cox 2 gene;
(vi) up to 30kb of an upstream gene sequence of the transcriptional start of a mammalian Ki67 gene;
(vii) an E4TF1 motif (-87 to -78) and an Sp-1 site (-129 to -121) and optionally a tandem repressor element CDE/CHR(-53 to -49/-39 to -35) of a mammalian Stk6 gene;
(viii) up to 5kb of 5' flanking region of a mammalian Hsp70 gene;
(ix) p53 recognition sequence repeats of a mammalian PIG3 gene; and
(x) up to 11.8kb of the 5' promoter region and 18 kb of a mammalian SOD2 gene and the 3'region of a mammalian SOD2 gene.

## Patentansprüche

1. Nukleinsäure-Konstrukt, umfassend
(i) eine Nukleinsäure-Sequenz einer Promotor-Region von Hmox-1, umfassend 16,5 kb stromaufwärts und 8 kb stromabwärts, gerechnet von der Transkriptions-Startstelle, eines Säuger-Hmox-1-Gens; und
(ii) eine Nukleinsäure-Sequenz, umfassend eine Transkriptions-Startstelle; und
(iii) wenigstens eine oder mehrere Nukleinsäure-Sequenz(en), die in der Lage ist/sind, als Matrize für ein definiertes RNS-Transkript zu wirken, und die ein Read-Out in Form eines ausscheidbaren Proteins und/oder eines Mittels liefert, das in der Lage ist, histologisch nachgewiesen zu werden.

2. Nukleinsäure-Sequenz nach Anspruch 1, worin die Nukleinsäure-Sequenz, die in der Lage ist, als Matrize für ein definiertes RNS-Transkript oder eine Reporter-Sequenz zu wirken, gewählt ist, um ein passendes Read-Out von Gen-Expression durch Assay entweder des Transkripts oder des kodierten Translations-Produkt-Poylpeptids zu liefern.

3. Nukleinsäure-Konstrukt nach Anspruch 2, worin die Transkription der Reporter-Sequenz nachgewiesen wird durch irgendeines oder mehr der folgenden:
(i) Assay des RNS-Transkripts der Reporter-Sequenz; oder
(ii) Assay eines Polypeptid-Translations-Produkts der Reporter-Sequenz.

4. Nukleinsäure-Konstrukt nach Anspruch 1, worin die Nukleinsäure-Sequenz einer Promotor-Region von Hmox-1 von einer Transkriptions-Startstelle operativ isoliert wird entweder durch (i) eine Nukleotid-Isolator-Sequenz, die auf einer ihrer Seiten flankiert ist von Nukleinsäure-Sequenzen, die von einer Site-spezifischen Rekombinase erkannt werden; oder (ii) durch Einbau des Promotors derart, dass er im Hinblick auf die Nukleinsäure-Sequenz, die eine Transkriptions-Startstelle umfasst, und die Nukleinsäure-Sequenz, die in der Lage ist, als Matrize für ein definiertes RNS-Transkript zu dienen, invertiert wird, und worin der invertierte Promotor zwischen Nukleinsäure-Sequenzen angeordnet ist, die von einer Site-spezifischen Rekombinase erkannt werden.

5. Nukleinsäure-Konstrukt nach Anspruch 4, worin die Rekombinase-Erkennungsstellen in der Weise angeordnet sind, dass die Nukleotid-Isolator-Sequenz entfernt wird oder die Orientierung der invertierten Promotor-Sequenz in Gegenwart der Rekombinase umgekehrt wird.

6. Nukleinsäure-Konstrukt nach einem der beiden Ansprüche 4 oder 5, weiter umfassend eine Nukleinsäure-Sequenz, die einen Gewebe-spezifischen Promotor umfasst, der operativ an ein Gen geknüpft ist, das für eine Kodierungs-Sequenz für die Site-spezifische Rekombinase kodiert.

7. Nukleinsäure-Konstrukt nach irgendeinem der Ansprüche 4 bis 6, worin das Site-spezifische Rekombinase-System das bakteriophage Pl-cre-Iox-System, das Gebrauch von zwei IoxP-Stellen macht, oder ein Bakterien-FLP-System ist

8. Wirtszelle, transfiziert mit einem Nukleinsäure-Konstrukt gemäß irgendeinem vorangehenden Anspruch, welches ein Protein exprimiert, das durch das Nukleinsäure-Konstrukt kodiert wird.

9. Wirtszelle nach Anspruch 8, die einer weiteren Transgenese unterworfen wird, worin die Transgenese die Einführung eines zusätzlichen Gens oder von Genen oder einer für ein Protein kodierenden Nukleinsäure-Sequenz oder Sequenzen ist.

10. Transgenes, nicht-menschliches Tier, einschließend ein Nukleinsäure-Konstrukt nach irgendeinem der Ansprüche 1 bis 7, worin Zellen des nicht-menschlichen Tieres ein Protein exprimieren, das durch das Nukleinsäure-Konstrukt kodiert wird.

11. Transgenes, nicht-menschliches Tier nach Anspruch 10, das einer weiteren Transgenese unterworfen wird, worin die Transgenese die Einführung eines weiteren Gens oder von Genen oder einer für ein Protein kodierende Nukleinsäure-Sequenz oder Sequenzen ist.

12. Verwendung eines Nukleinsäure-Konstrukts nach irgendeinem der Ansprüche 1 bis 7 für den Nachweis eines Gen-Aktivierungs-Ereignisses, das aus einem toxischen Insult in einer Leber- oder Nierenzelle *in vitro* resultiert und gegebenenfalls worin das Gen-Aktivierungs-Ereignis signalisiert wird durch Expression einer Reporter-Sequenz, deren Translationsprodukt mittels einer Epitop-Tag-Peptid-Sequenz identifiziert wird.

13. Verwendung nach Anspruch 12, worin die Nukleinsäure-Sequenz in der Lage ist, als Matrize für eine definierte Reporter-Sequenz oder RNS-Transkription zu wirken, und/oder Translationsprodukte heterolog gegenüber der Zelle sind, in der die Reporter-Sequenz exprimiert wird.

14. Verfahren zum Screenen, Chrakterisieren oder Überwachen von zellulären Stress-Antworten, einschließend eine Hmox-1-Gen-Aktivierung, in einer Leber- oder Nierenzelle oder -zelllinie entsprechend einem der beiden Ansprüche 8 oder 9 oder in der Leber oder in der Niere eines nicht-menschlichen transgenen Tiers entsprechend einem der beiden Ansprüche 10 oder 11, umfassend den Schritt, dass man die Zelle, Zelllinie oder das transgene nicht-menschliche Tier einem toxikologisch induzierten Stress aussetzt und ein Reporter- oder Read-Out-Produkt bzw. Produkte überwacht.

15. Wirtszelle nach einem der beiden Ansprüche 8 oder 9, welche weiter transfiziert ist mit einem oder mehreren zusätzlichen Nukleinsäure-Konstrukt(en), umfassend:
(a) eine Promotor-Region eines Gens oder Satzes von Genen, die gewählt sind aus der Gruppe, die besteht aus einem p21-, Metallothionein-1A-, PUMA-, Gclc-, Cox2-, Ki67-, Stk6-, Hsp-, PIG3- und SOD2-Gen und deren Expression in Antwort auf eine nachteilige extrazelluläre oder intrazelluläre Änderung der zellulären Umgebung modifiziert ist, die gewählt ist aus der Gruppe, die besteht aus
(i) Störungen des homöostatischen Zustands von DNS in der Zelle;
(ii) Änderungen in Beantwortung eines oxidativen Stress einer Zelle;
(iii) Änderungen, die hepatotoxischen Stress hervorrufen;
(iv) Stimuli, die eine zelluläre Apoptose triggern;
(v) Verabreichung von Chemikalien, therapeutischen Mitteln oder anderen xenobiotischen Mitteln; und
(vi) Krankheitszuständen, gleich ob natürlich, induziert oder modellmäßig aufgesetzt; und
(b) Nukleinsäure-Sequenz, die eine Transkriptions-Startstelle umfasst; und
(c) wenigstens eine oder mehrere Nukleinsäure-Sequenz(en), die in der Lage ist/sind, als Matrize für ein definiertes RNS-Transkript zu wirken und ein Read-Out in Form eines ausscheidbaren Proteins und/oder eines Mittels zu liefern, das in der Lage ist, histologisch nachgewiesen zu werden.

16. Wirtszelle nach Anspruch 15, worin die Promotor-Region irgendeine oder mehrere der folgenden Promotor-Sequenz(en) umfasst:
(i) bis zu 5 kb von einer Region unmittelbar in 5'-Richtung zu der Transkriptions-Startstelle eines Säuger-p21-Gens;
(ii) bis zu 26 kb eines stromaufwärts gelegenen Segments eines Säuger-Metallothionein-1A-Gens;
(iii) bis zu 16 kb einer stromaufwärts gelegenen Gen-Sequenz und ein 5 kb Segment einer stromabwärts gelegenen Sequenz eines Säuger-PUMA-Gens;
(iv) bis zu 16 kb einer stromaufwärts gelegenen Gen-Sequenz eines Säuger-Gclc-Gens;
(v) eine bis zu 3,5 kb Sequenz stromaufwärts der Transkriptions-Startstelle und ein weiteres 3 kb Segment von den kodierenden Sequenzen eines Säuger-cox-2-Gens;
(vi) bis zu 30 kb einer stromaufwärts gelegenen Gen-Sequenz des Transkriptions-Starts eines Säuger-Ki67-Gens;
(vii) ein E4TFI-Motiv (-87 bis -78) und eine Sp-1-Stelle (-129 bis -121) und gegebenenfalls ein Tandem-Repressor-Element CDE/CHR (-53 bis -49/-39 bis - 35) eines Säuger-Stk6-Gens;
(viii) bis zu 5 kb einer in 5'-Richtung flankierenden Region eines Säuger-Hsp70-Gens;
(ix) p53-Erkennungs-Sequenz-Repeats eines Säuger-PIG3-Gens; und
(x) bis zu 11,8 kb der 5'-Promotor-Region und 18 kb eines Säuger-SOD2-Gens und der 3'-Region eines Säuger-SOD2-Gens.

17. Transgenes, nicht-humanes Tier nach einem der beiden Ansprüche 10 oder 11, weiter einschließend ein oder mehrere Nukleinsäure-Konstrukt(e) umfassend:
(a) eine Promotor-Region eines Gens oder Satzes von Genen, die gewählt sind aus der Gruppe, die besteht aus einem p21-, Metallothionein-1A-, PUMA-, Gclc-, Cox2-, Ki67-, Stk6-, Hsp-, PIG3- und SOD2-Gen und deren Expression in Antwort auf eine nachteilige extrazelluläre oder intrazelluläre Änderung der zellulären Umgebung modifiziert ist, die gewählt ist aus der Gruppe, die besteht aus
(i) Störungen des homöostatischen Zustands von DNS in der Zelle;
(ii) Änderungen in Beantwortung eines oxidativen Stress einer Zelle;
(iii) Änderungen, die hepatotoxischen Stress hervorrufen;
(iv) Stimuli, die eine zelluläre Apoptose triggern;
(v) Verabreichung von Chemikalien, therapeutischen Mitteln oder anderen xenobiotischen Mitteln; und
(vi) Krankheitszuständen, gleich ob natürlich, induziert oder modellmäßig aufgesetzt; und
(b) Nukleinsäure-Sequenz, die eine Transkriptions-Startstelle umfasst; und
(c) wenigstens eine oder mehrere Nukleinsäure-Sequenz(en), die in der Lage ist/sind, als Matrize für ein definiertes RNS-Transkript zu wirken und ein Read-Out in Form eines ausscheidbaren Proteins und/oder eines Mittels zu liefern, das in der Lage ist, histologisch nachgewiesen zu werden.

18. Transgenes, nicht-humanes Tier entsprechend Anspruch 17, worin die Promotor-Region irgendeine oder mehrere der folgenden Promotor-Sequenzen umfasst:
(i) bis zu 5 kb von einer Region unmittelbar in 5'-Richtung zu der Transkriptions-Startstelle eines Säuger-p21-Gens;
(ii) bis zu 26 kb eines stromaufwärts gelegenen Segments eines Säuger-Metallothionein-1A-Gens;
(iii) bis zu 16 kb einer stromaufwärts gelegenen Gen-Sequenz und ein 5 kb Segment einer stromabwärts gelegenen Sequenz eines Säuger-PUMA-Gens;
(iv) bis zu 16 kb einer stromaufwärts gelegenen Gen-Sequenz eines Säuger-Gclc-Gens;
(v) eine bis zu 3,5 kb große Sequenz stromaufwärts der Transkriptions-Startstelle und ein weiteres 3 kb Segment von den kodierenden Sequenzen eines Säuger-cox-2-Gens;
(vi) bis zu 30 kb einer stromaufwärts gelegenen Gen-Sequenz des Transkriptions-Starts eines Säuger-Ki67-Gens;
(vii) ein E4TFI-Motiv (-87 bis -78) und eine Sp-1-Stelle (-129 bis -121) und gegebenenfalls ein Tandem-Repressor-Element CDE/CHR (-53 bis -49/-39 bis -35) eines Säuger-Stk6-Gens;
(viii) bis zu 5 kb einer in 5'-Richtung flankierenden Region eines Säuger-Hsp70-Gens;
(ix) p53-Erkennungs-Sequenz-Repeats eines Säuger-PIG3-Gens; und
(x) bis zu 11,8 kb der 5'-Promotor-Region und 18 kb eines Säuger-SOD2-Gens und der 3'-Region eines Säuger-SOD2-Gens.

19. Verfahren gemäß Anspruch 14, worin die Zellen oder Zelllinien oder nicht-menschlichen transgenen Tiere transfiziert sind mit oder tragen ein Hmox-1-Nukleinsäure-Konstrukt und wenigstens ein weiteres davon verschiedenes Nukleinsäure-Konstrukt, umfassend:
(a) eine Promotor-Region eines Gens oder Satzes von Genen, die gewählt sind aus der Gruppe, die besteht aus einem p21-, Metallothionein-1A-, PUMA-, Gclc-, Cox2-, Ki67-, Stk6-, Hsp-, PIG3- und SOD2-Gen und deren Expression in Antwort auf eine nachteilige extrazelluläre oder intrazelluläre Änderung der zellulären Umgebung modifiziert ist, die gewählt ist aus der Gruppe, die besteht aus
(i) Störungen des homöostatischen Zustands von DNS in der Zelle;
(ii) Änderungen in Beantwortung eines oxidativen Stress einer Zelle;
(iii) Änderungen, die hepatotoxischen Stress hervorrufen;
(iv) Stimuli, die eine zelluläre Apoptose triggern;
(v) Verabreichung von Chemikalien, therapeutischen Mitteln oder anderen xenobiotischen Mitteln; und
(vi) Krankheitszuständen, gleich ob natürlich, induziert oder modellmäßig aufgesetzt; und
(b) Nukleinsäure-Sequenz, die eine Transkriptions-Startstelle umfasst; und
(c) wenigstens eine oder mehrere Nukleinsäure-Sequenz(en), die in der Lage ist/sind, als Matrize für ein definiertes RNS-Transkript zu wirken und ein Read-Out in Form eines ausscheidbaren Proteins und/oder eines Mittels zu liefern, das in der Lage ist, histologisch nachgewiesen zu werden;
so dass eine Vielzahl von verschiedenen Reporter- oder Read-Out-Produkt(en) gleichzeitig überwacht werden können.

20. Verfahren nach Anspruch 19, worin die Promotor-Region irgendeine oder mehrere der folgenden Promotor-Sequenzen umfasst:
(i) bis zu 5 kb von einer Region unmittelbar in 5'-Richtung zu der Transkriptions-Startstelle eines Säuger-p21-Gens;
(ii) bis zu 26 kb eines stromaufwärts gelegenen Segments eines Säuger-Metallothionein-1A-Gens;
(iii) bis zu 16 kb einer stromaufwärts gelegenen Gen-Sequenz und ein 5 kb Segment einer stromabwärts gelegenen Sequenz eines Säuger-PUMA-Gens;
(iv) bis zu 16 kb einer stromaufwärts gelegenen Gen-Sequenz eines Säuger-Gclc-Gens;
(v) eine bis zu 3,5 kb Sequenz stromaufwärts der Transkriptions-Startstelle und ein weiteres 3 kb Segment von den kodierenden Sequenzen eines Säuger-cox-2-Gens;
(vi) bis zu 30 kb einer stromaufwärts gelegenen Gen-Sequenz des Transkriptions-Starts eines Säuger-Ki67-Gens;
(vii) ein E4TF1-Motiv (-87 bis -78) und eine Sp-1-Stelle (-129 bis -121) und gegebenenfalls ein Tandem-Repressor-Element CDE/CHR (-53 bis -49/-39 bis - 35) eines Säuger-Stk6-Gens;
(viii) bis zu 5 kb einer in 5'-Richtung flankierenden Region eines Säuger-Hsp70-Gens;
(ix) p53-Erkennungs-Sequenz-Repeats eines Säuger-PIG3-Gens; und
(x) bis zu 11,8 kb der 5'-Promotor-Region und 18 kb eines Säuger-SOD2-Gens und der 3'-Region eines Säuger-SOD2-Gens.

## Revendications

1. Produit de construction d'acide nucléique comprenant (i) une séquence d'acide nucléique d'une région promoteur de Hmox-1 comprenant 16,5 kb en amont et 8 kb en aval du site de démarrage de transcription d'un gène Hmox-1 de mammifère et (ii) une séquence d'acide nucléique comprenant un site de démarrage de transcription et (iii) au moins une ou plusieurs séquences d'acide nucléique capables d'agir comme matrice pour un produit de transcription d'ARN défini et de fournir une lecture sous la forme d'une protéine excrétable et/ou d'un agent capable d'être détecté histologiquement.

2. Séquence d'acide nucléique selon la revendication 1, dans laquelle la séquence d'acide nucléique capable d'agir comme matrice pour un produit de transcription d'ARN défini ou une séquence reporter définie est choisie pour fournir une lecture commode de l'expression génique par l'intermédiaire d'un essai soit du produit de transcription soit du polypeptide produit de traduction codé.

3. Produit de construction d'acide nucléique selon la revendication 2, dans lequel une transcription de la séquence reporter est détectée par un ou plusieurs des essais suivantes :
(i) essai du produit de transcription d'ARN de la séquence reporter ou ;
(ii) essai d'un produit de traduction de polypeptide de la séquence reporter.

4. Produit de construction d'acide nucléique selon la revendication 1, dans lequel la séquence d'acide nucléique d'une région promoteur de Hmox-1 est isolée fonctionnellement d'un site de démarrage de transcription soit (i) par une séquence isolateur nucléotidique qui est flanquée sur l'un et l'autre côté par des séquences d'acide nucléique reconnues par une recombinase spécifique du site, soit (ii) par insertion du promoteur de telle sorte qu'il est inversé par rapport à la séquence d'acide nucléique comprenant un site de démarrage de transcription et la séquence d'acide nucléique capable d'agir comme matrice pour un produit de transcription d'ARN défini et dans lequel le promoteur inversé est positionné entre des séquences d'acide nucléique reconnues par une recombinase spécifique du site.

5. Produit de construction d'acide nucléique selon la revendication 4, dans lequel les sites de reconnaissance de recombinase sont arrangés d'une manière telle que la séquence isolateur nucléotidique est délétée ou que l'orientation de la séquence promoteur inversée est inversée en présence de la recombinase.

6. Produit de construction d'acide nucléique selon l'une ou l'autre des revendications 4 ou 5, comprenant en outre une séquence d'acide nucléique comprenant un promoteur spécifique de tissu lié de façon fonctionnelle à un gène codant pour une séquence codante pour la recombinase spécifique du site.

7. Produit de construction d'acide nucléique selon l'une quelconque des revendications 4 à 6, dans lequel le système de recombinase spécifique du site est le système cre-lox du bactériophage P1 utilisant deux sites *lox*P ou un système FLIP bactérien.

8. Cellule hôte transfectée par un produit de construction d'acide nucléique selon l'une quelconque des revendications précédentes, qui exprime une protéine codée par le produit de construction d'acide nucléique.

9. Cellule hôte selon la revendication 8 qui est soumise à une nouvelle transgenèse, la transgenèse étant l'introduction d'un gène supplémentaire ou de gènes supplémentaires ou d'une séquence ou de séquences d'acide nucléique codant pour une protéine.

10. Animal non humain transgénique comprenant un produit de construction d'acide nucléique selon l'une quelconque des revendications 1 à 7, dans lequel des cellules de l'animal non humain expriment une protéine codée par le produit de construction d'acide nucléique.

11. Animal non humain transgénique selon la revendication 10, qui est soumis à une nouvelle transgenèse, la transgenèse étant l'introduction d'un gène supplémentaire ou de gènes supplémentaires ou d'une séquence ou de séquences d'acide nucléique codant pour une protéine.

12. Utilisation d'un produit de construction d'acide nucléique selon l'une quelconque des revendications 1 à 7 pour la détection d'un évènement d'activation de gène résultant d'une agression toxique dans une cellule hépatique ou rénale *in vitro* et facultativement dans laquelle l'évènement d'activation de gène est signalé par l'expression d'une séquence reporter dont le produit de traduction est identifié au moyen d'une séquence peptidique d'étiquette épitopique.

13. Utilisation selon la revendication 12, dans laquelle la séquence d'acide nucléique capable d'agir comme matrice pour une séquence reporter définie ou des produits de transcription et/ou de traduction d'ARN définis sont hétérologues à la cellule dans laquelle la séquence reporter est exprimée.

14. Procédé de criblage pour caractériser ou surveiller des réponses au stress cellulaire mettant en jeu une activation du gène Hmox-1 dans une cellule ou lignée cellulaire hépatique ou rénale selon l'une ou l'autre des revendications 8 ou 9 ou dans le foie ou le rein d'un animal transgénique non humain selon l'une ou l'autre des revendications 10 ou 11 comprenant l'exposition de la cellule, de la lignée cellulaire ou de l'animal non humain transgénique à un stress induit toxicologiquement et la surveillance du ou des produits reporter ou de lecture.

15. Cellule hôte selon l'une ou l'autre des revendications 8 ou 9 encore transfectée par un ou plusieurs produits de construction d'acide nucléique supplémentaires comprenant :
(a) une région promoteur d'un gène ou ensemble de gènes choisi dans le groupe constitué par un gène p21, métallothionéine 1A, PUMA, Gclc, Cox2, Ki67, Stk6, Hsp, PIG3 et SOD2 et dont l'expression est modifiée en réponse à un changement extracellulaire ou intracellulaire défavorable dans l'environnement cellulaire choisi dans le groupe constitué par :
(i) des perturbations de l'état homéostatique de l'ADN dans la cellule ;
(ii) des changements en réponse à un stress oxydant d'une cellule ;
(iii) des changements qui provoquent un stress hépatotoxique ;
(iv) des stimuli qui déclenchent une apoptose cellulaire ;
(v) l'administration de produits chimiques, de produits thérapeutiques ou autres agents xénobiotiques ; et
(vi) divers états de maladie qu'ils soient naturels, induits ou modélisés ; et
(b) une séquence d'acide nucléique comprenant un site de démarrage de transcription ; et
(c) au moins un ou plusieurs séquences d'acide nucléique capables d'agir comme matrice pour un produit de transcription d'ARN défini et de fournir une lecture sous la forme d'une protéine excrétable et/ou d'un agent capable d'être détecté histologiquement.

16. Cellule hôte selon la revendication 15, dans laquelle la région promoteur comprend l'une quelconque ou plusieurs des séquences promoteurs suivantes :
(i) jusqu'à 5 kb provenant d'une région immédiatement en 5' par rapport au site de démarrage de transcription d'un gène p21 de mammifère ;
(ii) jusqu'à 26 kb d'un segment en amont d'un gène de métallothionéine 1A de mammifère ;
(iii) jusqu'à 16 kb d'une séquence génique en amont et un segment de 5 kb de séquence en aval d'un gène PUMA de mammifère ;
(iv) jusqu'à 16 kb d'une séquence génique en amont d'un gène Gclc de mammifère ;
(v) jusqu'à 3,5 kb de séquence en amont à partir du site de démarrage de transcription et un autre segment de 3 kb à partir des séquences codantes d'un gène cox 2 de mammifère ;
(vi) jusqu'à 30 kb d'une séquence génique en amont du démarrage de transcription d'un gène Ki67 de mammifère ;
(vii) un motif E4TF1 (-87 à -78) et un site Sp-1 (-129 à -121) et facultativement un élément répresseur tandem CDE/CHR (-53 à -49/-39 à -35) d'un gène Stk6 de mammifère ;
(viii) jusqu'à 5 kb d'une région flanquante en 5' d'un gène Hsp70 de mammifère ;
(ix) des répétitions de la séquence de reconnaissance p53 d'un gène PIG3 de mammifère ; et
(x) jusqu'à 11,8 kb de la région promoteur en 5' et 18 kb d'un gène SOD2 de mammifère et la région 3' d'un gène SOD2 de mammifère.

17. Animal non humain transgénique selon l'une ou l'autre des revendications 10 ou 11 comprenant en outre un ou plusieurs produits de construction d'acide nucléique comprenant :
(a) une région promoteur d'un gène ou ensemble de gènes choisi dans le groupe constitué par un gène p21, métallothionéine 1A, PUMA, Gclc, Cox2, Ki67, Stk6, Hsp, PIG3 et SOD2 et dont l'expression est modifiée en réponse à un changement extracellulaire ou intracellulaire défavorable dans l'environnement cellulaire choisi dans le groupe constitué par :
(i) des perturbations de l'état homéostatique de l'ADN dans la cellule ;
(ii) des changements en réponse au stress oxydant d'une cellule ;
(iii) des changements qui provoquent un stress hépatotoxique ;
(iv) des stimuli qui déclenchent une apoptose cellulaire ;
(v) une administration de produits chimiques, de produits thérapeutiques ou autres agents xénobiotiques ; et
(vi) des états de maladie qu'ils soient naturels, induits ou modélisés ; et
(b) une séquence d'acide nucléique comprenant un site de démarrage de transcription ; et
(c) au moins une ou plusieurs séquences d'acide nucléique capables d'agir comme matrice pour un produit de transcription d'ARN défini et de fournir une lecture sous la forme d'une protéine excrétable et/ou d'un agent capable d'être détecté histologiquement.

18. Animal non humain transgénique selon la revendication 17, dans lequel la région promoteur comprend un quelconque ou plusieurs des séquences promoteurs suivantes :
(i) jusqu'à 5 kb à partir d'une région immédiatement en 5' par rapport au site de démarrage de transcription d'un gène p21 de mammifère ;
(ii) jusqu'à 26 kb d'un segment en amont d'un gène de métallothionéine 1A de mammifère ;
(iii) jusqu'à 16 kb d'une séquence génique en amont et un segment de 5 kb d'une séquence en aval d'un gène PUMA de mammifère ;
(iv) jusqu'à 16 kb d'une séquence génique en amont d'un gène Gclc de mammifère ;
(v) jusqu'à 3,5 kb d'une séquence en amont du site de démarrage de transcription et un autre segment de 3 kb à partir des séquences codantes d'un gène cox 2 de mammifère ;
(vi) jusqu'à 30 kb d'une séquence génique en amont du démarrage de transcription d'un gène Ki67 de mammifère ;
(vii) un motif E4TF1 (-87 à -78) et un site Sp-1 (-129 à - 121) et facultativement un élément répresseur tandem CDE/CHR (-53 à -49/-39 à -35) d'un gène Stk6 de mammifère ;
(viii) jusqu'à 5 kb d'une région flanquante en 5' d'un gène Hsp70 de mammifère ;
(ix) des répétitions de la séquence de reconnaissance p53 d'un gène PIG3 de mammifère ; et
(x) jusqu'à 11,8 kb de la région promoteur en 5' et 18 kb d'un gène SOD2 de mammifère et la région 3' d'un gène SOD2 de mammifère.

19. Procédé selon la revendication 14, dans lequel des cellules ou des lignées cellulaires ou des animaux transgéniques non humains sont transfectés par ou portent un produit de construction d'acide nucléique Hmox-1 et au moins un autre produit de construction d'acide nucléique différent comprenant :
(a) une région promoteur d'un gène ou ensemble de gènes choisi dans le groupe constitué par un gène p21, métallothionéine 1A, PUMA, Gclc, Cox2, Ki67, Stk6, Hsp, PIG3 et SOD2 et dont l'expression est modifiée en réponse à un changement extracellulaire ou intracellulaire défavorable dans l'environnement cellulaire choisi dans le groupe constitué par :
(i) des perturbations dans l'état homéostatique de l'ADN dans la cellule ;
(ii) des changements en réponse au stress oxydant d'une cellule ;
(iii) des changements qui provoquent un stress hépatotoxique ;
(iv) des stimuli qui déclenchent une apoptose cellulaire ;
(v) l'administration de produits chimiques, de produits thérapeutiques ou autres agents xénobiotiques ; et
(vi) des états de maladie qu'ils soient naturels, induits ou modélisés ; et
(b) une séquence d'acide nucléique comprenant un site de démarrage de transcription ; et
(c) au moins une ou plusieurs séquences d'acide nucléique capables d'agir comme matrice pour un produit de transcription d'ARN défini et de fournir une lecture sous la forme d'une protéine excrétable et/ou d'un agent capable d'être détecté histologiquement, de telle sorte que divers différents produit(s) reporter ou de lecture peuvent être surveillés simultanément.

20. Procédé selon la revendication 19, dans lequel la région promoteur comprend l'une quelconque ou plusieurs des séquences promoteurs suivantes :
(i) jusqu'à 5 kb provenant d'une région immédiatement en 5' par rapport au site de démarrage de transcription d'un gène p21 de mammifère ;
(ii) jusqu'à 26 kb d'un segment en amont d'un gène de métallothionéine 1A de mammifère ;
(iii) jusqu'à 16 kb d'une séquence génique en amont et un segment de 5 kb de séquence en aval d'un gène PUMA de mammifère ;
(iv) jusqu'à 16 kb d'une séquence génique en amont d'un gène Gclc de mammifère ;
(v) jusqu'à 3,5 kb de séquence en amont du site de démarrage de transcription et un autre segment de 3 kb provenant des séquences codantes d'un gène cox 2 de mammifère ;
(vi) jusqu'à 30 kb d'une séquence génique en amont du démarrage de transcription d'un gène Ki67 de mammifère ;
(vii) un motif E4TF1 (-87 à -78) et un site Sp-1 (-129 à -121) et facultativement un élément répresseur en tandem CDE/CHR (-53 à -49/-39 à -35) d'un gène Stk6 de mammifère ;
(viii) jusqu'à 5 kb d'une région flanquante en 5' d'un gène Hsp70 de mammifère ;
(ix) des répétitions de la séquence de reconnaissance p53 d'un gène PIG3 de mammifère ; et
(x) jusqu'à 11,8 kb de la région promoteur en 5' et 18 kb d'un gène SOD2 de mammifère et de la région 3' d'un gène SOD2 de mammifère.


### SEQUENCE LISTING

<110> CXR Biosciences Limited
   Roslin Institute (Edinburgh)
<120> Detection of Cellular Stress
<130> LPB/P107151WO
<140> PCT/GB2005/002779
   <141> 2005-07-15
<150> GB0415963.8
   <151> 2004-07-16
<160> 10
<170> Patent In version 3.3
<210> 1
   <211> 21
   <212> DNA
   <213> Artificial
<220>
   <223> Oligonucleotide sequence of primer
<400> 1
   gacaccagac caactggtaa t 21
<210> 2
   <211> 18
   <212> DNA
   <213> Artificial
<220>
   <223> Oligonucleotide sequence of primer
<400> 2
   gcatcgagcg taataagc 18
<210> 3
   <211> 30
   <212> DNA
   <213> Artificial
<220>
   <223> Oligonucleotide primer sequence
<400> 3
   ttcatagaat tcgactccaa cccatgaaac 30
<210> 4
   <211> 35
<212> DNA
   <213> Artificial
<220>
   <223> Oligonucleotide sequence primer
<400> 4
   tcgatcaagc ttgtctgcca gtgcacctaa cctgg 35
<210> 5
   <211> 33
   <212> DNA
   <213> Artificial
<220>
   <223> Oligonucleotide primer sequence
<400> 5
   ttcatagaat tcggtgcact ggccaggaag tcc 33
<210> 6
   <211> 33
   <212> DNA
   <213> Artificial
<220>
   <223> Oligonucleotide primer sequence
<400> 6
   cagtacaagc ttggagttgg atccctagta agg 33
<210> 7
   <211> 32
   <212> DNA
   <213> Artificial
<220>
   <223> Oligonucleotide primer sequence
<400> 7
   gtacaggaat tccttactag ggatccaact cc 32
<210> 8
   <211> 31
   <212> DNA
   <213> Artificial
<220>
   <223> Oligonucleotide primer sequence
<400> 8
   cagtacaagc ttcccactcc ttcaccgatc c 31
<210> 9
   <211> 30
   <212> DNA
   <213> Artificial
<220>
   <223> Oligonucleotide primer sequence
<400> 9
   tgacgtgaat tcgggcgcgc cctcttaacg 30
<210> 10
   <211> 39
   <212> DNA
   <213> Artificial
<220>
   <223> Oligonucleotide primer sequence
<400> 10
   tcacgtaagc ttctcgagct ctgaatgtct ggatatcgc 39